(19) Europäisches Patentamt
European Patent Office
Office européen des brevets

(11) Veröffentlichungsnummer: **0 387 194 B1**

(12) # EUROPÄISCHE PATENTSCHRIFT

(45) Veröffentlichungstag der Patentschrift: **18.01.95**

(51) Int. Cl.[6]: **C07F 9/38**, A61K 31/66

(21) Anmeldenummer: **90810150.4**

(22) Anmeldetag: **27.02.90**

(54) **N-substituierte Aminoalkandiphosphonsäuren.**

(30) Priorität: **08.03.89 CH 866/89**

(43) Veröffentlichungstag der Anmeldung:
**12.09.90 Patentblatt 90/37**

(45) Bekanntmachung des Hinweises auf die Patenterteilung:
**18.01.95 Patentblatt 95/03**

(84) Benannte Vertragsstaaten:
**AT BE CH DE DK ES FR GB GR IT LI LU NL SE**

(56) Entgegenhaltungen:
**EP-A- 0 177 443**
**EP-A- 0 272 208**
**WO-A-88/00829**
**DE-A- 3 623 397**

(73) Patentinhaber: **CIBA-GEIGY AG**
**Klybeckstrasse 141**
**CH-4002 Basel (CH)**

(72) Erfinder: **Jaeggi, Knut A., Dr.**
**General Guisan-Strasse 44**
**CH-4054 Basel (CH)**

EP 0 387 194 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft N-substituierte Aminoalkandiphosphonsäuren der Formel I

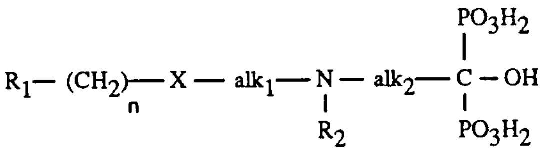

(I),

Worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ Wasserstoff oder einen einwertigen aliphatischen Rest bedeutet, und ihre Salze, Verfahren zur Herstellung der erfindungsgemässen Verbindungen, diese enthaltende pharmazeutische Präparate und ihre Verwendung als Arzneimittelwirkstoffe.

Der aromatische Rest $R_1$ ist beispielsweise ein mono- oder bicyclischer Aryl-, wie Phenyl-, Naphthyl- oder Indanylrest, oder ein 1 oder 2 N- Atome, 1 O- oder S- Atom, 1 N-und 1 O- Atom oder 1 N- und 1 S-Atom aufweisender Heteroaryl-, wie Imidazolyl-, Thiazolyl-, Oxazolyl- oder insbesondere Pyridyl-, Pyrimidinyl- oder Pyridazinylrest.

Die genannten Aryl- bzw. Heteroarylreste können substituiert, insbesondere durch Niederalkyl, Niederalkoxy, Niederalkylendioxy, Hydroxy, Trifluormethyl und/oder Halogen substituiert, wie mono-, di- oder trisubstituiert, insbesondere mono- oder disubstituiert, sein.

Einwertige aliphatische Reste sind beispielsweise Niederalkyl- oder Niederalkenylreste, zweiwertige aliphatische Reste beispielsweise Niederalkylenreste.

Gegebenenfalls oxydiertes Thio ist Thio, Sulfinyl oder Sulfonyl, insbesondere Thio oder Sulfonyl.

Gegebenenfalls aliphatisch substituiertes Imino ist beispielsweise $C_1$-$C_4$-Alkylimino, wie Methylimino, Aethylimino oder Propylimino.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise $C_1$-$C_7$-Alkyl, vorzugsweise $C_1$-$C_4$-Alkyl, wie Methyl, Aethyl, Propyl, Isopropyl oder Butyl, kann aber auch Isobutyl, Sekundärbutyl, Tertiärbutyl oder eine Pentyl-, Hexyl- oder Heptylgruppe sein.

Niederalkenyl ist beispielsweise $C_2$-$C_4$-Alkenyl, wie Vinyl, Allyl oder But-2-enyl, kann aber auch eine $C_5$-$C_7$-Alkenyl-, wie Pentenyl-, Hexenyl- oder Heptenylgruppe sein.

Niederalkylen ist beispielsweise $C_1$-$C_7$-Alkylen, im Falle von $alk_1$ insbesondere $C_2$-$C_6$-Alkylen, wie Aethylen, 1,3-Propylen, 1,4-Butylen oder 1,5-Pentylen, und im Falle von $alk_2$ insbesondere $C_1$-$C_4$-Alkylen, wie Aethylen oder in zweiter Linie Methylen oder 1,3-Propylen.

Niederalkoxy ist beispielsweise $C_1$-$C_7$-Alkoxy, vorzugsweise $C_1$-$C_4$-Alkoxy, wie Methoxy, Aethoxy, Propyloxy, Isopropyloxy oder Butyloxy, kann aber auch Isobutyloxy, Sekundärbutyloxy, Tertiärbutyloxy oder eine Pentyloxy-, Hexyloxy- oder Heptyloxygruppe sein.

Niederalkylendioxy ist beispielsweise Methylendioxy oder Aethylendioxy.

Halogen ist beispielsweise Halogen der Atomnummer bis und mit 35, wie Chlor oder Fluor, ferner Brom.

Salze von Verbindungen der Formel I sind beispielsweise deren Salze mit pharmazeutisch verwendbaren Basen, wie nicht- toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Aethyl- oder Diäthylamin, Mono-, Di- oder Tri-(hydroxyniederalkyl)aminen, wie Aethanol-, Diäthanol- oder Triäthanolamin, Tris-(hydroxymethyl)-methylamin oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. Tetrabutylammoniumhydroxid. Umfasst sind sowohl vollständige als auch partielle Salze, d.h. Salze mit 1, 2, 3 oder 4, vorzugsweise 2, Aequivalenten Base pro Mol Säure der Formel I.

Die Verbindungen der Formel I und ihre Salze weisen wertvolle pharmakologische Eigenschaften auf. Insbesondere besitzen sie eine ausgeprägte regulierende Wirkung auf den Calcium- Stoffwechsel von

Warmblütern. So bewirken sie an der Ratte eine ausgeprägte Hemmung der Knochenresorption, die sich sowohl in der Versuchsanordnung gemäss Acta Endocrinol. 78, 613-24(1975) als auch anhand des PTH-induzierten Anstieges des Serumcalciumspiegels nach subcutaner Applikation in Dosen von etwa 0,01 bis etwa 1.0 mg/kg, als auch im TPTX-(Thyroparathyroidectomised)-Rattenmodell anhand der durch Vitamin $D_3$ ausgelösten experimentellen Hypercalzämie nach Gabe von Dosen von etwa 0,0005 bis etwa 0,5 mg/kg s.c. und teilweise auch p.o. zeigen lässt. Ebenso wird die durch Walker-256-Tumore induzierte Tumorhypercalzämie nach peroraler Verabreichung von etwa 1,0 bis etwa 100 mg/kg gehemmt. Ferner zeigen sie in der Adjuvansarthritis der Ratte in der Versuchsanordnung nach Newbold, Brit. J. Pharmacology 21, 127(1963) sowie nach Kaibara et al., J. Exp. Med. 159, 1388-96 (1984) in Dosen von etwa 0,01 bis etwa 1,0 mg/kg s.c. eine deutliche Hemmung des Fortschreitens chronisch- arthritischer Prozesse. Im Vordergrund stehen dabei die Indikationen tumorinduzierte Hypercalzämie, Knochenmatastasen und morbus Paget.

Die Verbindungen der Formel I und ihre Salze sind deshalb vorzüglich geeignet als Arzneimittelwirkstoffe für die Behandlung von Erkrankungen, die mit Störungen des Calcium- Stoffwechsels in Verbindung gebracht werden können, beispielsweise entzündliche Prozesse in Gelenken, degenerative Prozesse im Gelenkknorpel, von Osteoporosis, Periodontitis, Hyperparathyreoidismus, und von Calciumablagerungen in Blutgefässen oder in prothetischen Implantaten.

Die Erfindung betrifft beispielsweise Verbindungen der Formel I, worin $R_1$, n, X, $alk_1$ und $alk_2$ die angegebenen Bedeutungen haben und $R_2$ für einen einwertigen aliphatischen Rest steht, und ihre Salze.

Die Erfindung betrifft in erster Linie Verbindungen der Formel I, worin $R_1$ einen unsubstituierten oder durch Niederalkyl, Niederalkoxy, Niederalkylendioxy, Hydroxy, Halogen und/oder Trifluormethyl mono-, di- oder trisubstituierten, insbesondere mono- oder disubstituierten Phenyl-, Naphthyl-, Imidazolyl-, Thiazolyl-Oxazolyl-, Pyridyl-, Pyrimidinyl-oder Pyridazinylrest bedeutet, n für 0, 1, 2 oder 3 steht, X Oxy, Thio, Sulfinyl, Sulfonyl, Imino oder Niederalkylimino darstellt, $alk_1$ Niederalkylen bedeutet, $R_2$ für Wasserstoff, Niederalkyl oder Niederalkenyl steht und $alk_2$ Niederalkylen bedeutet, und ihre Salze, insbesondere ihre Salze.

Die Erfindung betrifft vor allem einerseits Verbindungen der Formel I, worin $R_1$ einen unsubstituierten oder durch $C_1$-$C_4$-Alkyl, wie Methyl, $C_1$-$C_4$-Alkoxy, wie Methoxy, $C_1$-$C_4$-Alkylendioxy, wie Methylendioxy oder Aethylendioxy, Hydroxy, Halogen der Atomnummer bis und mit 35, wie Fluor oder Chlor, und/oder Trifluormethyl mono- oder disubstituierten Phenylrest bedeutet, n für 0 oder in zweiter Linie für 1 oder 2 steht, X Oxy, Thio, Sulfonyl, Imino oder $C_1$-$C_4$-Alkylimino, wie Methylimino oder Aethylimino, darstellt, $alk_1$ $C_2$-$C_6$-Alkylen, wie Aethylen, 1,3-Propylen, 1,4-Butylen, 1,5-Pentylen oder 1,6-Hexylen, bedeutet, $R_2$ für Wasserstoff oder $C_1$-$C_4$-Alkyl, wie Methyl, Aethyl, Propyl oder Butyl, steht und $alk_2$ $C_2$-$C_4$-Alkylen, wie Aethylen, darstellt, und ihre Salze, insbesondere ihre pharmazeutisch verwendbaren Salze.

Die Erfindung betrifft vor allem andrerseits Verbindungen der Formel I, worin $R_1$ einen unsubstituierten oder durch $C_1$-$C_4$-Alkyl, wie Methyl, und/oder unsubstituiertes oder durch $C_1$-$C_4$-Alkyl, wie Methyl, $C_1$-$C_4$-Alkoxy, wie Methoxy, $C_1$-$C_4$-Alkylendioxy, wie Methylendioxy oder Aethylendioxy, Hydroxy, Halogen der Atomnummer bis und mit 35, wie Fluor oder Chlor, und/oder Trifluormethyl mono- oder disubstituiertes Phenyl substituierten Pyridyl- oder Pyrimidinylrest bedeutet, n für 0 oder in zweiter Linie für 1 oder 2 steht, X Oxy, Thio, Imino oder $C_1$-$C_4$-Alkylimino, wie Methylimino oder Aethylimino, darstellt, $alk_1$ $C_2$-$C_6$-Alkylen, wie Aethylen, 1,3-Propylen, 1,4-Butylen, 1,5-Pentylen oder 1,6-Hexylen, bedeutet, $R_2$ für Wasserstoff oder $C_1$-$C_4$-Alkyl, wie Methyl, Aethyl, Propyl oder Butyl, steht und $alk_2$ $C_2$-$C_4$-Alkylen, wie Aethylen, darstellt, und ihre Salze, insbesondere ihre pharmazeutisch verwendbaren Salze.

Die Erfindung betrifft vor allem beispielsweise Verbindungen der Formel I, worin $R_1$ einen unsubstituierten oder durch $C_1$-$C_4$-Alkyl, wie Methyl, $C_1$-$C_4$-Alkoxy, wie Methoxy, $C_1$-$C_4$-Alkylendioxy, wie Methylendioxy oder Aethylendioxy, Hydroxy, Halogen der Atomnummer bis und mit 35, wie Fluor oder Chlor, und/oder Trifluormethyl mono- oder disubstituierten Phenyl-, Naphthyl-, Pyridyl- oder Pyrimidinylrest bedeutet, n für 0, 1 oder 2 steht, X Oxy, Thio, Sulfonyl, Imino oder $C_1$-$C_4$-Alkylimino, wie Methylimino oder Aethylimino, darstellt, $alk_1$ $C_2$-$C_6$-Alkylen, wie Aethylen, 1,3-Propylen, 1,4-Butylen, 1,5-Pentylen oder 1,6-Hexylen, bedeutet, $R_2$ für $C_1$-$C_4$-Alkyl, wie Methyl, Aethyl, Propyl oder Butyl, steht und $alk_2$ $C_2$-$C_4$-Alkylen, wie Aethylen, darstellt, und ihre Salze, insbesondere ihre pharmazeutisch verwendbaren Salze.

Die Erfindung betrifft insbesondere Verbindungen der Formel I, worin $R_1$ unsubstituiertes oder durch $C_1$-$C_4$-Alkyl, wie Methyl, $C_1$-$C_4$-Alkoxy, wie Methoxy, Hydroxy, Halogen der Atomnummer bis und mit 35, wie Fluor oder Chlor, oder Trifluormethyl substituiertes Phenyl oder unsubstituiertes Pyridyl oder Pyrimidinyl bedeutet, n für 0 steht, X Oxy, Thio, Sulfonyl, Imino oder Methylimino bedeutet, $alk_1$ $C_2$-$C_6$-Alkylen, wie Aethylen, 1,3-Propylen, 1,4-Butylen, 1,5-Pentylen oder 1,6-Hexylen, darstellt, $R_2$ Wasserstoff oder $C_1$-$C_4$-Alkyl, wie Methyl, Aethyl, Propyl oder Butyl, bedeutet und $alk_2$ Aethylen ist, und ihre Salze, insbesondere ihre pharmazeutisch verwendbaren Salze.

Die Erfindung betrifft insbesondere beispielsweise Verbindungen der Formel I, worin $R_1$ unsubstituiertes oder durch $C_1$-$C_4$-Alkyl, wie Methyl, $C_1$-$C_4$-Alkoxy, wie Methoxy, Hydroxy, Halogen der Atomnummer bis und mit 35, wie Fluor oder Chlor, oder Trifluormethyl substituiertes Phenyl oder unsubstituiertes Pyridyl oder Pyrimidinyl bedeutet, n für 0 steht, X Oxy, Thio, Sulfonyl, Imino oder Methylimino bedeutet, $alk_1$ $C_2$-$C_6$-Alkylen, wie Aethylen, 1,3-Propylen, 1,4-Butylen, 1,5-Pentylen oder 1,6-Hexylen, darstellt, $R_2$ $C_1$-$C_3$-Alkyl, wie Methyl oder Aethyl, bedeutet und $alk_2$ Aethylen ist, und ihre Salze, insbesondere ihre pharmazeutisch verwendbaren Salze.

Die Erfindung betrifft in allererster Linie einerseits Verbindungen der Formel I, worin $R_1$ unsubstituiertes oder durch $C_1$-$C_4$-Alkyl, wie Methyl, $C_1$-$C_4$-Alkoxy, wie Methoxy, Hydroxy, oder Halogen der Atomnummer bis und mit 35, wie Fluor oder Chlor, substituiertes Phenyl bedeutet, n für 0 steht, X Oxy oder Thio bedeutet, $alk_1$ $C_2$-$C_4$-Alkylen, wie Aethylen, 1,3-Propylen oder 1,4-Butylen, $R_2$ $C_1$-$C_3$-Alkyl, wie Methyl, oder in zweiter Linie Wasserstoff bedeutet und $alk_2$ Aethylen ist, und ihre Salze, insbesondere ihre pharmazeutisch verwendbaren Salze.

Die Erfindung betrifft in allererster Linie andrerseits Verbindungen der Formel I, worin $R_1$ unsubstituiertes oder durch $C_1$-$C_4$-Alkyl, wie Methyl, oder Phenyl substituiertes Pyridyl oder Pyrimidyl bedeutet, n für 0 steht, X unsubstituiertes oder durch $C_1$-$C_4$-Alkyl, wie Methyl oder Aethyl, substituiertes Imino, Oxy oder Thio bedeutet, $alk_1$ $C_2$-$C_4$-Alkylen, wie Aethylen, 1,3-Propylen oder 1,4-Butylen, $R_2$ $C_1$-$C_3$-Alkyl, wie Methyl, oder in zweiter Linie Wasserstoff bedeutet und $alk_2$ Aethylen ist, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Die Erfindung betrifft ferner ein auf an sich bekannten Methoden beruhendes Verfahren zur Herstellung der erfindungsgemässen Verbindungen. Dieses ist dadurch gekennzeichnet, dass man

a) in einer Verbindung der Formel

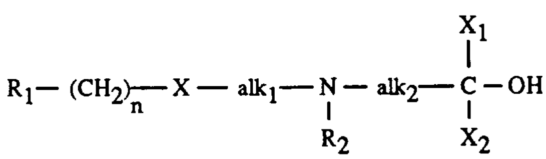 (II),

worin $R_1$, n, X, $alk_1$, $R_2$ und $alk_2$ die angegebenen Bedeutungen haben, $X_1$ eine funktionell abgewandelte und $X_2$ eine freie oder funktionell abgewandelte Phosphonogruppe bedeutet, funktionell abgewandeltes Phosphono $X_1$ und gegebenenfalls $X_2$ in die freie Phosphonogruppe überführt oder

b) Verbindungen der Formeln

$$R_1-(CH_2)_n-X-alk_1-Y_1 \quad (III)$$

und

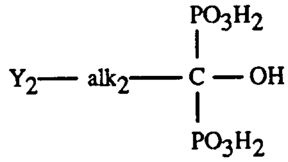 (IV),

worin einer der Reste $Y_1$ und $Y_2$ eine reaktionsfähige veresterte Hydroxygruppe Y und der andere eine Gruppe der Formel $-N(R_2)-H$ bedeutet, oder deren Salze oder unter reduzierenden Bedingungen Verbindungen der Formeln

$$R_1-(CH_2)_n-X-alk'_1=O \quad (IIIa)$$

und

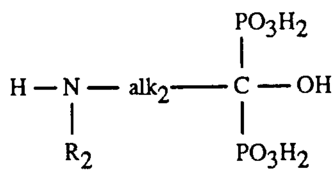

(IVa),

worin $alk_1'$ einen dem Rest $alk_1$ entsprechenden doppelt gebundenen Rest, z.B. einen entsprechend substituierten Niederalkanylylidenrest, darstellt, miteinander umsetzt oder

c) eine Verbindung der Formel

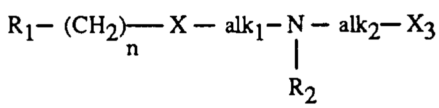

(V),

worin $X_3$ Carboxy, Carbamyl oder Cyano bedeutet, mit einem Phosphorylierungsmittel umsetzt, das Primärprodukt hydrolysiert und in einem ausgehend von Verbindungen der Formel V, worin $X_3$ Cyano oder Carbamyl ist, erhaltenen Zwischenprodukt der Formel

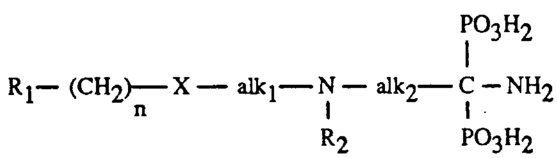

(VI),

bzw. einem Salz davon die Aminogruppe durch Behandlung mit salpetriger Säure durch Hydroxy ersetzt oder

d) Verbindungen der Formeln

$$R_1-(CH_2)_n-Y_3 \quad (VII)$$

und

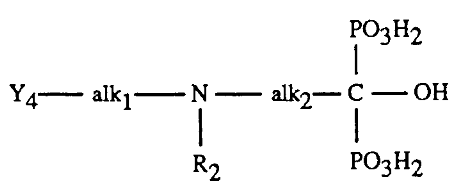

(VIII),

worin $Y_3$ eine Gruppe der Formel $-X_0-H$ und $Y_4$ reaktionsfähiges verestertes Hydroxy Y bedeutet oder, sofern n von 0 verschieden ist, $Y_3$ eine Gruppe Y und $Y_4$ eine Gruppe $-X_0-H$ darstellt, wobei $X_0$ Oxy, Thio oder gegebenenfalls aliphatisch substituiertes Imino X oder intermediär geschütztes Imino $-NR_0-$, worin $R_0$ eine übliche Aminoschutzgruppe ist, bedeutet und Y reaktionsfähiges verestertes Hydroxy darstellt, oder deren Salze oder zur Herstellung von Verbindungen der Formel I, worin X eine gegebenenfalls aliphatisch substituierte Iminogruppe darstellt, unter reduzierenden Bedingungen Verbindungen der Formeln

$$R_1—(CH_2)_{n-1}—CH=O \quad (VIIa)$$

und

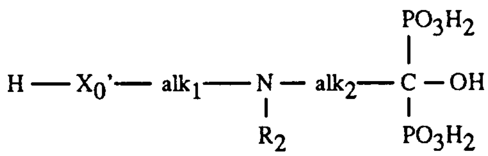
(VIIIa),

bzw.

$$R_1-(CH_2)_n—X_0'—H \quad (VIIb)$$

und

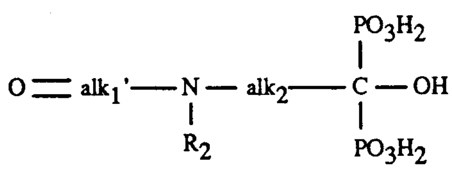
(VIIIb),

worin $R_1$, n, $alk_1$, $R_2$ und $alk_2$ die angegebenen Bedeutungen haben und $X'_0$ gegebenenfalls aliphatisch substituiertes Imino X oder intermediär geschütztes Imino $-NR_0-$ darstellt, wobei $R_0$ eine übliche Aminoschutzgruppe ist, miteinander umsetzt, jeweils die Aminoschutzgruppe $R_0$, sofern vorhanden abspaltet und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Geeignete Aminoschutzgruppen $R_0$ sind beispielsweise gegebenenfalls substituierte $\alpha$-Aralkyl-, wie Benzyl-, oder Benzyloxycarbonylgruppen, veresterte oder verätherte Hydroxymethylgruppen, wie Pivaloyloxymethyl, Methoxymethyl, 2-Chloräthoxymethyl oder Benzyloxymethyl, Tetrahydropyranyl oder Triniederalkylsilyl, wie Trimethylsilyl. Die Schutzgruppe wird beispielsweise durch Umsetzung der zu

schützenden Verbindung mit einem entsprechenden Halogenderivat bzw. mit Chlorjodmethan ($Cl\text{-}CH_2I$), einem Alkalimetall-, z.B. Natriumpivalat, -methanolat, -1,2-dichloräthanolat oder -benzylalkoholat bzw. mit Dihydropyran, eingeführt. Geschütztes Imino ist dementsprechend beispielsweise Silylimino, wie Trimethylsilylimino, kann aber auch Phenyl-, Diphenyl- oder Triphenylniederalkylimino, wie Benzylimino, Diphenylimino oder Triphenylimino, sein.

Gemäss der Verfahrensvariante a) in Phosphono zu überführende funktionell abgewandelte Phosphonogruppen liegen beispielsweise in einer Esterform, insbesondere einer Diesterform der Formel $-P(=O)(OR)_2$ (IIa), vor, worin OR veräthertes Hydroxy, insbesondere Niederalkoxy, Niederalkanoyloxyniederalkoxy oder eine gegebenenfalls durch Niederalkyl, Niederalkoxy, Halogen, Trifluormethyl und/oder Hydroxy substituierte Phenoxy- oder $\alpha$-Phenylniederalkoxygruppe oder Silyloxy, wie Triniederalkylsilyloxy, bedeutet.

Die Ueberführung von funktionell abgewandelten in freie Phosphonogruppen erfolgt in üblicher Weise, wie durch Hydrolyse, beispielsweise in Gegenwart einer Mineralsäure, wie Salz- oder Schwefelsäure, bei etwa 80°C bis etwa 110°C, z.B. in der Siedehitze, oder durch Umsetzung mit einem Triniederalkylhalogensilan, z.B. mit Trimethylchlorsilan oder insbesondere Trimethyljodsilan oder Trimethylbromsilan, vorzugsweise in Methylenchlorid im Temperaturbereich von etwa 0°C bis etwa 40°C, und anschliessende Behandlung mit Wasser. $\alpha$-Phenylniederalkylester können ferner durch Hydrogenolyse, beispielsweise Umsetzung mit Wasserstoff in Gegenwart eines Hydrierungskatalysators, wie eines Nickel- oder Edelmetallkatalysators, z.B. von Palladium auf Kohle, vorzugsweise in einem Niederalkanol unter normalen Temperatur- und Druckbedingungen, in Verbindungen der Formel I überführt werden.

Die Ausgangsstoffe der Formel II können beispielsweise hergestellt werden, indem man eine Verbindung der Formel

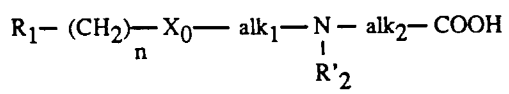

(IIb),

wobei $X_0$ Oxy, Thio oder gegebenenfalls aliphatisch substituiertes Imino X oder intermediär geschütztes Imino $-NR_0-$, worin $R_0$ eine übliche Aminoschutzgruppe ist, und $R_2'$ eine Gruppe $R_2$ oder eine Aminoschutzgruppe $R_0$ bedeutet, oder vorzugsweise das Anhydrid oder Säurechlorid derselben mit einem entsprechenden Phosphorigsäuretriester der Formel $P(OR)_3$ (IIc), beispielsweise bei 0°C bis etwa 60°C, zu einer Verbindung der Formel

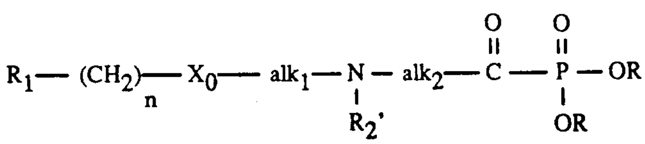

(IId),

kondensiert und diese mit einem Phosphorigsäurediester der Formel $H\text{-}P(=O(OR)_2$ (IIe) bzw. $P(OH)(OR)_2$ (IIf) in Gegenwart eines Diniederalkylamins, z.B. von Diäthylamin, oder eines Alkalimetallniederalkanolates, z.B. von Natriummethanolat, zur entsprechenden Verbindung der Formel

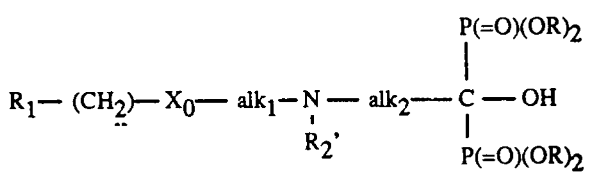

(IIg),

weiterumsetzt, gegebenenfalls die Aminoschutzgruppe(n) $R_0$ abspaltet und/oder gewünschtenfalls einen von von Wasserstoff verschiedenen Rest $R_2$ einführt. z.B. wie nachstehend unter b) beschrieben.

Ausgangsstoffe der Formel IIb können, soweit sie nicht bekannt sind, beispielsweise hergestellt werden, indem man eine entsprechende Verbindung der Formel $R_1\text{-}(CH_2)_n\text{-}X_0\text{-}alk_1\text{-}N(R_2')\text{-}H$ (IIh), worin $R_2'$ eine Gruppe $R_2$ oder eine Aminoschutzgruppe $R_0$ bedeutet, mit einer Verbindung der Formel $Y\text{-}alk_2\text{-}COOH$ (IIi), worin Y Halogen, wie Brom, ist oder zur Herstellung von Verbindungen IIb, worin $alk_2$ 1 2-

Niederalkylen, z.B. Aethylen, bedeutet, einer Verbindung der Formel $alk_0$-COOR (IIj), worin $alk_0$ Niederalk-1-enyl bedeutet, umsetzt, jeweils den erhaltenen Ester zur Säure hydrolysiert, diese, z.B. mittels Phosphorpentachlorid, anhydridisiert bzw. chloriert, eine gegebenenfalls vorhandene Aminoschutzgruppe abspaltet und erforderlichen- oder gewünschtenfalls die freigesetzte Aminogruppe aliphatisch substituiert.

Gemäss der Verfahrensvariante b) zu verwendende reaktive Ester III bzw. IV weisen als reaktionsfähige veresterte Hydroxygruppen beispielsweise ein Halogen-, wie Chlor-, Brom- oder Jodatom, oder eine Sulfonyloxygruppe, z.B.. Methansulfonyloxy oder p-Toluolsulfonyloxy, auf.

Die Umsetzung mit den genannten reaktiven Estern erfolgt beispielsweise in Gegenwart einer Base, wie eines Alkalimetall- oder Erdalkalimetallhydroxides, z.B. von Natriumhydoxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Aethers, z.B. von Isopropanol, Methyläthylketon, Dioxan oder Tetrahydrofuran.

Die Umsetzung mit Oxoverbindungen IIIa wird beispielsweise in Gegenwart eines Alkalimetallborhydrides, z.B. von Natriumcyanoborhydrid, oder insbesondere durch Behandeln mit Ameisensäure durchgeführt.

Die Ausgangsstoffe der Formel IV können beispielsweise hergestellt werden, indem man eine Verbindung der Formel $Y_2$-$alk_2$-$X_3$ (IVb) in üblicher Weise, beispielsweise in Chlorbenzol, mit phosphoriger Säure und Phosphortrichlorid bzw. mit Phosphorsäure und einem Überschuss an Phosphortribromid umsetzt und anschliessend hydrolytisch aufarbeitet. Ausgangsstoffe IVa können in analoger Weise durch Umsetzung von Verbindungen der Formel $R_0$-N($R_2$)-$alk_2$-$X_3$ (IVc) mit phosphoriger Säure und Phosphortrichlorid hergestellt werden, wobei $R_0$ eine übliche Aminoschutzgruppe bedeutet.

Als Phosphorylierungmittel für die Verfahrensvariante c) kommen beispielsweise Phosphortrioxid, Phosphortrihalogenide im Gemisch mit phosphoriger Säure oder Phosphorsäure, Phosphoroxychlorid oder Phosphorpentachlorid bzw. Phosphortrichlorid im Gemisch mit Chlor in Betracht. Bevorzugt ist Phosphortrioxid, welches vorzugsweise durch Umsetzung von Phosphortrichlorid mit phosphoriger Säure bzw. die Phosphorigsäurekomponente vorzugsweise durch Reaktion mit einem Ueberschuss von Phosphortrichlorid mit wasserhaltiger Phosphorsäure, z.B. mit handelsüblicher etwa 75%-iger bis 95%-iger, vorzugsweise etwa 85%-iger, Phosphorsäure, *in situ* gebildet wird. Die Umsetzung wird vorteilhaft unter Erwärmen, z.B. auf etwa 70 bis 120 °C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel VI mit salpetriger Säure erfolgt in üblicher Weise unter Freisetzung derselben in wässriger Lösung aus einem ihrer Salze, z.B. aus Natriumnitrit, durch Säurebehandlung, z.B. Einwirkung von Salzsäure, wobei intermediär ein entsprechendes instabiles Diazoniumsalz, z.B. -chlorid, gebildet wird, das unter Einführung der $\alpha$-Hydroxygruppe Stickstoff abspaltet.

Die Ausgangsstoffe der Formel V können, soweit sie nicht bekannt sind, beispielsweise hergestellt werden, indem man eine entsprechende Verbindung der Formel $R_1$-$(CH_2)_n$-$X_0$-$alk_1$-N($R_2$')-H(IIh), mit einer Verbindung der Formel Y-$alk_2$-$X_3$ (Va), worin Y Halogen, wie Brom, ist oder zur Herstellung von Verbindungen der Formel V, worin $alk_2$ 1,2-Niederalkylen, z.B. Aethylen, bedeutet, mit einer Verbindung der Formel $alk_0$-$X_3$ (Vb), worin $alk_0$ einen Niederalk-1-enylrest bedeutet, umsetzt, jeweils die Aminoschutzgruppe, sofern vorhanden, abspaltet und gewünschtenfalls jeweils das erhaltene Primärprodukt zur Säure hydrolysiert.

Gemäss der Verfahrensvariante d) zu verwendende reaktive Ester VII bzw. VIII weisen als reaktionsfähige veresterte Hydroxygruppen beispielsweise ein Halogen-, wie Chlor-, Brom- oder Jodatom, oder eine Sulfonyloxygruppe, z.B.. Methansulfonyloxy oder p-Toluolsulfonyloxy, auf.

Die Umsetzung mit den genannten reaktiven Estern erfolgt beispielsweise in Gegenwart einer Base, wie eines Alkalimetall- oder Erdalkalimetallhydroxides, z.B. von Natriumhydoxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Aethers, z.B. von Isopropanol, Methyläthylketon, Dioxan oder Tetrahydrofuran.

Ausgehend von Thiolen VII bzw. VIII oder Phenolen bzw. Thiophenolen VII kommen als basische Kondensationsmittel ebenso oder vorzugsweise Alkalimetallcarbonate, wie Natriumcarbonat oder Kaliumcarbonat, vorteilhaft in einem aliphatischen oder cycloaliphatischen Keton, z.B. in Aceton oder Cyclohexanon, in Betracht, wobei man vorzugsweise bei erhöhter Temperatur, z.B. im Temperaturbereich von etwa 50° bis etwa 100 °C, z.B. in der Siedehitze, arbeitet.

Die Umsetzung mit Oxoverbindungen VIIa bzw. VIIIb wird beispielsweise in Gegenwart eines Alkalimetalborhydrides, z.B. von Natriumcyanoborhydrid oder insbesondere durch Behandeln mit Ameisensäure durchgeführt. Insbesondere kann man ein Verbindung der Formel IVa, worin $R_2$ einen Rest $R_1$-($CH_2$)-$alk_1$- bedeutet, unter reduzierenden Bedingungen mit einem Niederalkanal, beispielsweise mit Formaldehyd, und Ameisensäure, oder in zweiter Linie mit einem reaktionsfähigen Ester eines Niederalkanols bzw. Niederalkenols in üblicher Weise, vorzugsweise in Gegenwart eines basischen Kondensationsmittels, wie eines Alkalimetallniederalkanolates, durch einen Niederalkylrest $R_2$ substituieren.

Die Ausgangsstoffe der Formel VII, VIIa und VIIb sind weitgehend bekannt. Ausgangsstoffe der Formeln VIII, VIIIa und VIIIb können beispielsweise hergestellt werden, indem man eine Verbindung der Formel Y-$alk_2$-$X_3$ (VIIId) in üblicher Weise, beispielsweise in Chlorbenzol, mit phosphoriger Säure und Phosphortrichlorid bzw. mit Phosphorsäure und einem Ueberschuss an Phosphortribromid umsetzt, anschliessend hydrolytisch aufarbeitet und die erhaltene Verbindung der Formel

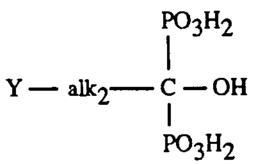

(VIIIe)

in üblicher Weise mit einer Verbindung der Formeln $Y_4$-$alk_1$-N($R_2$')-H (VIIIf), H-$X_0$-$alk_1$-N($R_2$')-H (VIIIg) oder O = $alk_1$'-N($R_2$')-H (VIIIh) weiterumsetzt.

Die Freisetzung intermediär geschützter Reste, z.B. Abspaltung einer Aminoschutzgruppe $R_0$, erfolgt in üblicher Weise, beispielsweise durch Hydrogenolyse, z.B. in Gegenwart von Platin- oder Palladiumkatalysatoren, bzw. Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salzoder Essigsäure.

Verfahrensgemäss erhältliche Verbindungen können in üblicher Weise in andere Verbindungen der Formel I überführt werden.

So kann man in Verbindungen der Formel I, worin $R_2$ Wasserstoff bedeutet, in üblicher Weise durch Umsetzung mit einem reaktiven Ester der Formel $R_2$-Y (IX), worin Y reaktionsfähiges verestertes Hydroxy, beispielsweise ein Halogen-, wie Chlor-, Bromoder Jodatom, oder eine Sulfonyloxygruppe, z.B. Methansulfonyloxy oder p-Toluolsulfonyloxy, bedeutet, oder durch Umsetzung mit einen aliphatischen Aldehyd oder Keton der Formel $R_2$ = O (IXa) unter reduzierenden Bedingungen,einen einwertigen aliphatischen Rest $R_2$ einführen. Insbesondere kann man zur Herstellung von Verbindungen der Formel I, worin $R_2$ einen einwertigen aliphatischen Rest bedeutet, und ihrer Salze

e) Verbindungen der Formeln

$R_2$-Y (IX)

und

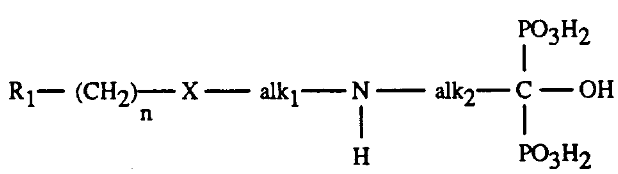

(Ia),

worin $R_1$, n, X, $alk_1$, $R_2$ und $alk_2$ die angegebenen Bedeutungen haben und Y für reaktionsfähiges verestertes Hydroxy bedeutet, oder ihre Salze oder unter reduzierenden Bedingungen Verbindungen der Formeln

$R_2$'' = O (IXa)

und

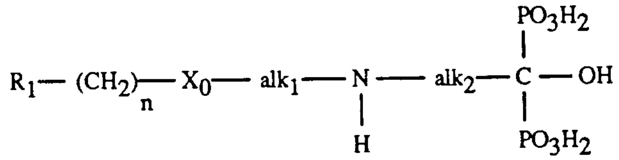

(Ia),

worin $R_1$, n, X, $alk_1$ und $alk_2$ die angegebenen Bedeutungen haben, $X_0$ Oxy, Thio oder gegebenenfalls aliphatisch substituiertes Imino X oder intermediär geschütztes Imino $-NR_0-$, worin $R_0$ eine übliche Aminoschutzgruppe ist, und $R_2''$ für einen dem Rest $R_2$ entsprechenden doppeltgebundenen Rest, z.B. für Niederalkyliden, steht, miteinander umsetzen.

Die Umsetzung mit den genannten reaktiven Estern (IX) erfolgt beispielsweise in Gegenwart einer Base, wie eines Alkalimetall- oder Erdalkalimetallhydroxides, z.B. von Natriumhydoxid, oder eines quaternären Ammoniumhydroxides, z.B. von Tetrabutylammoniumhydroxid, vorteilhaft in Gegenwart eines Lösungs- oder Verdünnungsmittels, z.B. eines Niederalkanols, Diniederalkylketons oder cycloaliphatischen Aethers, z.B. von Isopropanol, Methyläthylketon, Dioxan oder Tetrahydrofuran. Die Umsetzung mit Oxoverbindungen (IXa) wird beispielsweise in Gegenwart eines Alkalimetallborhydrides, z.B. von Natriumcyanoborhydrid, oder insbesondere durch Behandeln mit Ameisensäure durchgeführt. In einer bevorzugten Ausführungsform kann man eine entsprechende Verbindung der Formel Ia unter reduzierenden Bedingungen mit einem Niederalkanal, beispielsweise mit Formaldehyd, und Ameisensäure, durch einen Niederalkylrest $R_2$ substituieren.

Ferner kann man Verbindungen der Formel I, worin X Thio bedeutet, in üblicher Weise zu der entsprechenden Verbindung der Formel I, worin X Sulfinyl oder Sulfonyl ist, oxydieren, beispielsweise durch Behandlung mit einer anorganischen Peroxyverbindung, z.B. mit Wasserstoffperoxyd, Perschwefelsäure oder einer organischen Peroxyverbindung, wie Perbenzoesäure oder m-Chlorperbenzoesäure.

Auch kann man in den Rest $R_1$ von Verbindungen der Formel I Substituenten einführen, Niederalkyl beispielsweise durch Umsetzung mit einem Niederalkylhalogenid in Gegenwart von Aluminiumtrichlorid, Niederalkoxy beispielsweise durch Nitrieren, Reduktion der Nitrogruppe zur Aminogruppe, Diazotieren derselben und Behandeln des gebildeten Diazoniumsalzes mit dem entsprechenden Niederalkanol unter Erwärmen und Halogen beispielsweise durch Behandeln mit Chlor oder Brom, vorteilhaft in Gegenwart einer Lewissäure, z.B. von Eisen-III-chlorid. Man kann aber auch Halogen durch Trifluormethyl ersetzen, beispielsweise durch Behandeln mit Trifluorjodmethan in Gegenwart von Kupferpulver oder Kupfer-I-jodid.

Ferner kann man Verbindungen der Formel I, worin X Imino ist, durch einen aliphatischen Rest substituieren, beispielsweise wie vorstehend für die Einführung eines von Wasserstoff verschiedenen Restes $R_2$ beschrieben.

Die neuen Verbindungen können, je nach Wahl der Ausgangsstoffe und Arbeitsweisen, in Form eines der möglichen Isomeren, z.B. je nach Anzahl der asymmetrischen Kohlenstoffatome als optische Isomere, wie in Form eines Enantiomeren, wie Antipoden, bzw. Diastereomeren, oder als Gemische derselben, wie Enantiomerengemische, z.B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung einer Verbindung der Formel 1 oder eines Anhydrides davon mit einer optisch aktiven Base bzw. mit einem optisch aktiven Alkohols und Trennung der erhaltenen diastereomeren Ester, z.B. auf Grund ihrer verschieden Löslichkeiten, in die Diastereomeren, aus denen die Enantiomeren durch Einwirkung geeigneter Mittel freigesetzt werden können. Racemate der Formel I können auch durch Umsetzung mit einer optisch aktiven Base in Gemische der diastereomeren Salze, und Trennung derselben in die Diastereomeren, aus denen die Enantiomeren in der jeweils üblichen Weise freigesetzt werden können, gespalten werden.

Für diesen Zweck übliche optisch aktive Basen sind z.B. optisch aktive Alkaloide, wie Chinin, Chinchonin, Brucin und dergl., oder insbesondere α-Phenyläthylamin.

Ferner können erhaltene salzbildende Verbindungen in an sich bekannter Weise in Salze überführt werden, z.B. durch Umsetzung einer Lösung der freien Verbindung in einem geeigneten Lösungsmittel oder Lösungsmittelgemisch mit einer entsprechenden Base oder mit einem geeigneten Ionenaustauscher.

Erhaltene Salze können in an sich bekannter Weise in die freien Verbindungen umgewandelt werden, z.B. durch Behandeln mit einer Säure, wie einer Mineralsäure, z.B. mit Salzsäure.

Erhaltene Salze können in an sich bekannter Weise in andere Salze überführt werden, z.B. durch Behandeln mit einer geeigneten Base, wie Natriumhydoxid oder Kaliumhydroxid, Ammoniak oder einem geeigneten Amin.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. Mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Zur parenteralen Verabreichung eignen sich in erster Linie wässrige Lösungen des Wirkstoffes in wasserlöslicher Form, z.B. in Form eines wasserlöslichen pharmazeutisch verwendbaren Salzes, ferner Suspensionen das Wirkstoffes, wie ölige Injektionssuspensionen, wobei man geeignete lipophile Lösungsmittel oder Vehikel, wie fette Oele, z.B. Sesamöl, oder synthetische Fettsäureester, z.B. Aethyloleat, ferner Triglyceride, verwendet oder wässrige Injektionssuspensionen, welche viskositätserhöhende Stoffe, z.B. Natriumcarboxymethylcellulose, Sorbit und/oder Dextran, und gegebenenfalls Stabilisatoren enthalten.

Die Erfindung betrifft weiterhin die Verwendung von Verbindungen der Formel I, zur Behandlung von auf Störungen des Calciumstoffwechsels zurückzuführenden Erkrankungen, vorzugsweise durch Bereitstellung von pharmazeutischen Präparaten. Die Dosierung der erfindungsgemässen Verbindung der Formel I kann von verschiedenen Faktoren, wie Applikationsweise, Spezies, Alter und/oder individuellem Zustand abhängen. Einzeldosen enthalten beispielsweise von etwa 0,01 bis etwa 0,1 mg, vorzugsweise 0,02 bis 0,08 mg bei parenteraler und etwa 0,2 bis etwa 2,5 mg, vorzugsweise 0,3 bis 1,5 mg, bei oraler Gabe jeweils pro Kilogramm Körpergewicht. Die bevorzugten Einzeldosen betragen somit etwa 0.5 bis 5.0 mg bei parenteraler und etwa 10 bis 100 mg bei oraler Applikation. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgen Beispiele dienen zur Illustration der Erfindung; Temperaturen sind in Celsiusgraden, Drucke in mbar angegeben.

<u>Beispiel 1</u>: 27,3 g (0,1 Mol) 3-[N-(3-Phenoxypropyl)-N-methyl-amino]-propionsäure-hydrochlorid werden mit 13,4 mi 85%-iger Phosphorsäure und 50 ml Chlorbenzol unter Rühren und Rückfluss auf 100° erhitzt.

Dann werden bei 100° 27 ml Phosphortrichlorid zugetropft, wobei Gasentwicklung eintritt. Das Reaktionsgemisch scheidet im Laufe von 30 Minuten eine dicke Masse ab. Man erhitzt noch 2 Stunden auf 100° und dekantiert dann das überstehende Chlorbenzol ab. Die zurückbleibende zähe Masse wird mit 100 ml 4-n Salzsäure 3 Stunden unter Rühren und Rückfluss zum Sieden erhitzt. Man filtriert heiss unter Kohlezusatz und verdünnt das Filtrat mit Aceton, wobei sich die 3-[N-(3-Phenoxypropyl)-N-methyl-amino]-1-hydroxy-propan-1,1-diphosphonsäure kristallin abscheidet; Smp. 198-200° (Zers.).

Das Ausgangsmaterial kann z.B. folgendermassen hergestellt werden:

23,75g (0,15Mol) N-(3-Phenoxypropyl)-N-methyl-amin werden in 50 ml Diäthyläther vorgelegt und unter Rühren allmählich mit 15,1 ml Acrylsäureäthylester versetzt. Unter leichtem Temperaturanstieg bildet sich eine Klare Lösung. Nach Stehenlassen über Nacht bei Raumtemperatur wird der Diäthyläther abdestilliert. Das zurückbleibende Oel stellt rohen 3-[N-(3-Phenoxypropyl)-N-methyl-amino]-propionsäureäthylester dar.

Der erhaltene Ester wird in 600 ml 4n- Salzsäure 24 Stunden zum Rückfluss erhitzt. Dann wird unter vermindertem Druck völlig eingedampft und der kristalline Rückstand mit Aceton verrieben. Nach Waschen, Abnutschen und Trocknen der Kristalle erhält man das 3-[N-(3-Phenoxypropyl)-N-methyl-amino]-propionsäure-hydrochlorid vom Smp. 127-128°.

Beispiel 2: In analoger Weise wie in Beispiel 1 beschrieben erhält man ausgehend von
3-{N-[2-(4-Methoxyphenoxy)äthyl]amino}-propionsäure-hydrochlorid ,Smp. 105-108°, die
3-{N-[2-(4-Methoxyphenoxy)äthyl]amino}-1-hydroxy-propan-1,1-diphosphonsäure, Smp.118-124°.

Beispiel 3: In analoger Weise wie in Beispiel 1 beschrieben erhält man ausgehend von
3-[N-(2-Phenoxyäthyl)-N-methyl-amino]-propionsäure-hydrochlorid vom Smp. 104-106° die
3-[N-(2-Phenoxyäthyl)-N-methyl-amino]-1-hydroxy-propan-1,1-diphosphonsäure, Smp. 125-130° (Zers.).

Beispiel 4: In analoger Weise wie in Beispiel 1 beschrieben erhält man ausgehend von
3-[N-(3-Phenoxypropyl)-N-äthyl-amino]-propionsäure-hydrochlorid vom Smp. 105-108° die
3-[N-(3-Phenoxypropyl)-N-äthyl-amino]-1-hydroxy-propan-1,1 -diphosphonsäure, Smp. 195-197° (Zers.).

Beispiel 5: In analoger Weise wie in Beispiel 1 beschrieben erhält man ausgehend von
3-[N-(3-Phenoxypropyl)-N-propyl-amino]-propionsäure-hydrochlorid (Oel) die
3-[N-(3-Phenoxypropyl)-N-propyl-amino]-1-hydroxy-propan-1,1 -diphosphonsäure, Smp. 197-199° (Zers.).

Beispiel 6: In analoger Weise wie in Beispiel 1 beschrieben erhält man ausgehend von
3-[N-(3-Phenoxypropyl)-N-butyl-amino]-propionsäure-hydrochlorid (Oel) die
3-[N-(3-Phenoxypropyl)-N-butyl-amino]- 1-hydroxy-propan-1,1-diphosphonsäure, Smp. 181-183° (Zers.).

Beispiel 7: In analoger Weise wie in Beispiel 1 beschrieben erhält man ausgehend von
3-{N-[3-(4-Methoxyphenoxy)propyl]-N-methyl-amino}-propionsäure-hydrochlorid vom Smp. 115-116° die
3-{N-[3-(4-Methoxyphenoxy)propyl]-N-methyl-amino}-1-hydroxy-propan-1,1-diphosphonsäure, Smp. 128-130° (Zers.).

Beispiel 8: In analoger Weise wie in Beispiel 1 beschrieben erhält man ausgehend von
3-{N-[2-(2-Methoxyphenoxy)äthyl]-N-methyl-amino}-propionsäure-hydrochlorid vom Smp. 147-149° unter gleichzeitiger Aetherspaltung an der Methoxygruppe die
3-{N-[2-(2-Hydroxyphenoxy)äthyl]-N-methyl-amino}-1-hydroxy-propan-1,1-diphosphonsäure, Smp. 141-145° (Zers.).

Beispiel 9: In analoger Weise wie in Beispiel 1 beschrieben erhält man ausgehend von
3-{N-[3-(4-Chlorphenoxy)propyl]-N-methyl-amino}-propionsäure-hydrochlorid vom Smp. 129-132° die
3-{N-[3-(4-Chlorphenoxy)propyl]-N-methyl-amino}-1-hydroxy-propan-1,1-diphosphonsäure, Smp. 155-162° (Zers.).

Beispiel 10: In analoger Weise wie in Beispiel 1 beschrieben erhält man ausgehend von
3-{{N-{3-[N'-(Pyrid-2-yl)-N'-methyl-amino]propyl}-N-methyl- amino}}-propionsäure-hydrochlorid (Harz) die
3-{{N-{3-[N'-(Pyrid-2-yl)-N'-methyl-amino]propyl}-N-methyl-amino}}-1-hydroxy-propan-1,1-diphosphonsäure. Dieses liegt als Phosphorsäureadditionssalz der Summenformel $C_{13}H_{25}N_3O_7P_2$ x $\frac{3}{4}$ $H_3PO_4$ x $H_2O$ vom Smp. 158-161° (Zers.) vor.

Beispiel 11: In analoger Weise wie in Beispiel 1 beschrieben erhält man ausgehend von
3-[N-(2-Phenylthioäthyl)-N-methyl-amino]-propionsäure-hydrochlorid vom Smp. 103-104° die
3-[N-(2-Phenylthioäthyl)-N-methyl-amino]-1-hydroxy-propan-1,1-diphosphonsäure, Smp. 165-168° (Zers.).

Beispiel 12: In analoger Weise wie in Beispiel 1 beschrieben erhält man ausgehend von
3-[N-(3-Phenoxypropyl)amino]-propionsäure-hydrochlorid vom Smp. 121-122° die
3-[N-(3-Phenoxypropyl)amino]-1-hydroxy-propan-1,1-diphosphonsäure, Smp. 148-155° (Zers.).

Beispiel 13: In analoger Weise wie in Beispiel 1 beschrieben erhält man ausgehend von
3-{N-[2-(4-Chlorphenoxy)äthyl]-N-methyl-amino}-propionsäure-hydrochlorid vom Smp. 176-179° die
3-{N-[2-(4-Chlorphenoxy)äthyl]-N-methyl-amino]-1-hydroxy-propan-1,1-diphosphonsäure, Smp. 216-218° (Zers.).

Beispiel 14: In analoger Weise wie in Beispiel 1 beschrieben erhält man ausgehend von
3-{N-[3-(4-Fluorphenoxy)propyl]-N-methyl-amino}-propionsäure-hydrochlorid vom Smp. 119-121° die
3-{N-[3-(4-Fluorphenoxy)propyl]-N-methyl-amino]-1-hydroxy-propan-1,1-diphosphonsäure, Smp. 165-172° (Zers.).

Beispiel 15: In analoger Weise wie in Beispiel 1 beschrieben erhält man ausgehend von
3-[N-(4-Phenoxybutyl)-N-methyl-amino]-propionsäure-hydrochlorid vom Smp. 114-116° die
3-[N-(4-Phenoxybutyl)-N-methyl-amino]-1-hydroxy-propan-1,1-diphosphonsäure, Smp. 153-156° (Zers.).

Beispiel 16: In analoger Weise wie in Beispiel 1 beschrieben erhält man ausgehend von
3-[N-(6-Phenoxyhexyl)-N-methyl-amino]-propionsäure-hydrochlorid vom Smp. 108-109° die
3-[N-(6-Phenoxyhexyl)-N-methyl-amino]-1-hydroxy-propan-1,1-diphosphonsäure, Smp. 183-187° (Zers.).

Beispiel 17: In analoger Weise wie in Beispiel 1 beschrieben kann man folgende Verbindungen herstellen:
3-[N-(3-Phenylthiopropyl)-N-methyl-amino]-1-hydroxy-propan-1,1-diphosphonsäure;
3-{N-[3-(3-Methylphenoxy)propyl]-N-methyl-amino}-1-hydroxy-propan-1,1-diphosphonsäure;
3-[N-(3-(4-Methoxyphenoxy)propylamino]-1-hydroxy-propan-1,1-diphosphonsäure;
3-[N-(4-(Phenylthiobutyl)-N-methyl-amino]-1-hydroxy-propan-1,1-diphosphonsäure;
3-{N-[2-(3-Trifluormethylphenylamino)äthyl]-N-methyl-amino}-1-hydroxy-propan-1,1-diphosphonsäure;
3-{N-[3-(2-Pyridylamino)propyl]-N-methyl-amino}-1-hydroxy-propan-1,1-diphosphonsäure;
3-{N-[3-(2-Pyrimidinylamino)propyl]-N-methyl-amino}-1-hydroxy-propan-1,1-diphosphonsäure und
3-[N-(2-(Benzolsulfonyl)äthyl]-N-methyl-amino]-1-hydroxy-propan-1,1-diphosphonsäure.

Beispiel 18: In analoger Weise wie in Beispiel 1 beschrieben erhält man ausgehend von
3-{N-[3-(3-Methylphenoxy)propyl]-N-methyl-amino]-propionsäure-hydrochlorid vom-Smp. 109-114° die
3-{N-[3-(3-Methylphenoxy)propyl]-N-methyl-amino}-1-hydroxy-propan-1,1-diphosphonsäure vom Smp. 152-158° (Zers.).

Beispiel 19: In analoger Weise wie in Beispiel 1 beschrieben kann man auch die 3-{{N-{3-[N'-(Pyrid-2-yl)-N'-äthyl-amino]propyl}-N-äthyl-amino}}-1-hydroxy-propan-1,1-diphosphonsäure herstellen.

Beispiel 20: 0,92 g 3-[N-(3-Phenoxypropyl)amino]-1-hydroxy-propan-1,1-diphosphonsäure werden mit 75 ml 98%-iger Ameisensäure und 0,5 ml 35%-iger wässriger Formaldehydlösung 20 Stunden unter Rühren zum Rückfluss erhitzt. Das Reaktionsgemisch wird untervermindertem Druck eingeengt und der Rückstand aus Aceton kristallisiert. Man erhält die 3-[N-(3-Phenoxypropyl)-N-methyl-amino]-1-hydroxy-propan-1,1-diphosphonsäure vom Smp. 198-200° (Zers.).

Beispiel 21: Tabletten, enthaltend je 50 mg 3-[N-(3-Phenoxypropyl)-N-methyl-amino]-1-hydroxy-propan-1,1-diphosphonsäure oder ein Salz, z.B. das Dinatriumsalz, davon können wie folgt hergestellt werden:

| Zusammensetzung (10000 Tabletten) | |
|---|---|
| Wirkstoff | 500,0 g |
| Lactose | 500,0 g |
| Kartoffelstärke | 325,0 g |
| Gelatine | 8,0 g |
| Talk | 60,0 g |
| Magnesiumstearat | 10,0 g |
| Siliciumdioxid (hochdispers) | 20,0 g |
| Aethanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat und das Siliciumdioxid zu und presst das Gemisch zu Tabletten von je 145,0 mg Gewicht und 50,0 mg Wirkstoffgehalt, die gewünschtenfalls mit

Teilkerben zur feineren Anpassung der Dosierung versehen sein können.

Beispiel 22: Lacktabletten, enthaltend je 100 mg 3-[N-(3-Phenoxypropyl)-N-methylamino]-1-hydroxy-propan-1,1-diphosphonsäure oder ein Salz, z.B. das Dinatriumsalz, davon können wie folgt hergestellt werden:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt Das Gemisch wird zu Tabletten (Gewicht: 280 mg) verpresst und diese mit einer Lösung der Hydroxypropyl-methylcellulose und des Schellacks in Methylenchlorid lackiert; Endgewicht der Lacktablette: 283 mg.

Beispiel 23: Gelatinesteckkapseln, enthaltend 100 mg Wirkstoff, z.B. 3-[N-(3-Phenoxypropyl)-N-methyl-amino]-1-hydroxy-propan-1,1-diphosphonsäure oder ein Salz, z.B. das Dinatriumsalz, davon, können z.B. folgendermassen hergestellt werden:

| Zusammensetzung (für 1000 Kapseln) | |
|---|---|
| Wirkstoff | 100,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird die mikrokristalline Zellulose durch ein Sieb mit einer Maschenweite von 0,9 mm hinzugesiebt. Dann wird erneut 10 Minuten innig vermischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

Beispiel 24: Eine 0,2%-ige Injektions- oder Infusionslösung von 3-[N-(3-Phenoxypropyl)-N-methyl-amino]-1-hydroxy-propan-1,1-diphosphonsäure oder eines ihrer Salze, z.B. ihr Dinatriumsalz, kann beispielsweise folgendermassen hergestellt werden:

| Zusammensetzung (für 1000 Ampullen) | |
|---|---|
| Wirkstoff | 5,0 g |
| Natriumchlorid | 22,5 g |
| Phosphatpuffer pH = 7.4 | 300,0 g |
| entmineralisiertes Wasser | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glas- oder Kunststoffampullen abgefüllt, die dann je 2,0 bzw. 5,0 mg Wirkstoff enthalten.

Beispiel 25: In analoger Weise wie in den Beispielen 21 bis 24 beschrieben kann man auch pharmazeutische Präparate, enthaltend eine andere Verbindung der Formel I gemäss einem der Beispiele 2 bis 20 herstellen.

**Patentansprüche**

**Patentansprüche für folgende Vertragsstaaten : AT, BE, CH, DE, DK, FR, GB, IT, LI, LU, NL, SE**

1. Verbindungen der Formel I

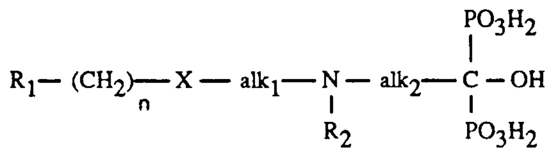

(I),

worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ Wasserstoff oder einen einwertigen aliphatischen Rest bedeutet, und ihre Salze.

2. Verbindungen gemäss Anspruch 1 der Formel I, worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ einen einwertigen aliphatischen Rest bedeutet, und ihre Salze.

3. Verbindungen gemäss Anspruch 1 der Formel I, worin $R_1$ einen unsubstituierten oder durch Niederalkyl, Niederalkoxy, Niederalkylendioxy, Hydroxy, Halogen und/oder Trifluormethyl mono-, di- oder trisubstituierten Phenyl-, Naphthyl-, Imidazolyl-, Thiazolyl- Oxazolyl-, Pyridyl-, Pyrimidinyl- oder Pyridazinylrest bedeutet, n für 0, 1, 2 oder 3 steht, X Oxy, Thio, Sulfinyl, Sulfonyl, Imino oder Niederalkylimino darstellt, $alk_1$ Niederalkylen bedeutet, $R_2$ für Wasserstoff, Niederalkyl oder Niederalkenyl steht und $alk_2$ Niederalkylen bedeutet, und ihre Salze, insbesondere ihre pharmazeutisch verwendbaren Salze.

4. Verbindungen gemäss Anspruch 1 der Formel I, worin $R_1$ einen unsubstituierten oder durch $C_1$-$C_4$-Alkyl, $C_1$-$C_4$-Alkcoxy, $C_1$-$C_4$-Alkylendioxy, Hydroxy, Halogen der Atomnummer bis und mit 35 und/oder Trifluormethyl mono- oder disubstituierten Phenylrest bedeutet, n für 0, 1 oder 2 steht, X Oxy, Thio, Sulfonyl, Imino oder $C_1$-$C_4$-Alkylimino darstellt, $alk_1$ $C_2$-$C_6$-Alkylen bedeutet, $R_2$ für Wasserstoff oder $C_1$-$C_4$-Alkyl steht und $alk_2$ $C_2$-$C_4$-Alkylen darstellt, und ihre Salze.

5. Verbindungen gemäss Anspruch 1 der Formel I, worin $R_1$ einen unsubstituierten oder durch $C_1$-$C_4$-Alkyl und/oder unsubstituiertes oder durch $C_1$-$C_4$-Alkyl, $C_1$-$C_4$-Alkoxy, $C_1$-$C_4$-Alkylendioxy, Hydroxy, Halogen der Atomnummer bis und mit 35 und/oder Trifluormethyl mono- oder disubstituiertes Phenyl substituierten Pyridyl- oder Pyrimidinylrest bedeutet, n für 0, 1 oder 2 steht, X Oxy, Thio, Imino oder $C_1$-$C_4$-Alkylimino darstellt, $alk_1$ $C_2$-$C_6$-Alkylen bedeutet, $R_2$ für Wasserstoff oder $C_1$-$C_4$-Alkyl steht und $alk_2$ $C_2$-$C_4$-Alkylen darstellt, und ihre Salze.

6. Verbindungen gemäss Anspruch 2 der Formel I, worin $R_1$ einen unsubstituierten oder durch $C_1$-$C_4$-Alkyl, $C_1$-$C_4$-Alkoxy, $C_1$-$C_4$-Alkylendioxy, Hydroxy, Halogen der Atomnummer bis und mit 35 und/oder Trifluormethyl mono- oder disubstituierten Phenyl-, Naphthyl-, Pyridyl- oder Pyrimidinylrest bedeutet, n für 0, 1 oder 2 steht, X Oxy, Thio, Sulfonyl, Imino oder $C_1$-$C_4$-Alkylimino darstellt, $alk_1$ $C_2$-$C_6$-Alkylen bedeutet, $R_2$ für $C_1$-$C_4$-Alkyl steht und $alk_2$ $C_2$-$C_4$-Alkylen darstellt, und ihre Salze.

7. Verbindungen gemäss Anspruch 1 der Formel I, worin $R_1$ unsubstituiertes oder durch $C_1$-$C_4$-Alkyl, $C_1$-$C_4$-Alkoxy, Hydroxy, Halogen der Atomnummer bis und mit 35 oder Trifluormethyl substituiertes Phenyl oder unsubstituiertes Pyridyl oder Pyrimidinyl bedeutet, n für 0 steht, X Oxy, Thio, Sulfonyl, Imino oder Methylimino bedeutet, $alk_1$ $C_2$-$C_6$-Alkylen darstellt, $R_2$ Wasserstoff oder $C_1$-$C_4$-Alkyl bedeutet und $alk_2$ Aethylen ist, und ihre Salze.

8. Verbindungen gemäss Anspruch 2 der Formel I, worin $R_1$ unsubstituiertes oder durch $C_1$-$C_4$-Alkyl, $C_1$-$C_4$-Alkoxy, Hydroxy, Halogen der Atomnummer bis und mit 35 oder Trifluormethyl substituiertes Phenyl oder unsubstituiertes Pyridyl oder Pyrimidinyl bedeutet, n für 0 steht, X Oxy, Thio, Sulfonyl, Imino oder

Methylimino bedeutet, $alk_1$ $C_2$-$C_6$-Alkylen darstellt, $R_2$ $C_1$-$C_3$-Alkyl bedeutet und $alk_2$ Aethylen ist, und ihre Salze.

**9.** Verbindungen gemäss Anspruch 1 der Formel I, worin $R_1$ unsubstituiertes oder durch $C_1$-$C_4$-Alkyl, $C_1$-$C_4$-Alkoxy oder Halogen der Atomnummer bis und mit 35 substituiertes Phenyl bedeutet, n für 0 steht, X Oxy oder Thio bedeutet, $alk_1$ $C_2$-$C_4$-Alkylen darstellt, $R_2$ $C_1$-$C_3$-Alkyl oder Wasserstoff bedeutet und $alk_2$ Aethylen ist, und ihre Salze.

**10.** Verbindungen gemäss Anspruch 1 der Formel I, worin $R_1$ unsubstituiertes oder durch $C_1$-$C_4$-Alkyl oder Phenyl substituiertes Pyridyl oder Pyrimidinyl bedeutet, n für 0 steht, X unsubstituiertes oder durch $C_1$-$C_4$-Alkyl substituiertes Imino, Oxy oder Thio bedeutet, $alk_1$ $C_2$-$C_4$-Alkylen bedeutet und $R_2$ $C_1$-$C_3$-Alkyl oder Wasserstoff bedeutet und $alk_2$ Aethylen ist, und ihre Salze.

**11.** 3-[N-(3-Phenoxypropyl)-N-methyl-amino]-1-hydroxy-propan-1,1-diphosphonsäure oder ein Salz davon.

**12.** 3-{N-[2-(4-Methoxyphenoxy)äthyl]amino}-1-hydroxy-propan-1,1 diphosphonsäure oder ein Salz davon.

**13.** 3-[N-(2-Phenoxyäthyl)-N-methyl-amino]-1-hydroxy-propan-1,1-diphosphonsäure oder ein Salz davon.

**14.** 3-[N-(2-Phenylthioäthyl)-N-methyl-amino]-1-hydroxy-propan-1,1-diphosphonsäure oder ein Salz davon.

**15.** 3-[N-(3-Phenoxypropyl)amino]-1-hydroxy-propan-1,1-diphosphonsäure oder ein Salz davon.

**16.** 3-[N-(3-Phenylthiopropyl)-N-methyl-amino]-1-hydroxy-propan-1,1-diphosphonsäure oder ein Salz davon.

**17.** 3-{N-[3-(4-Chlorphenoxy)propyl]-N-methyl-amino-1-hydroxy-propan-1,1-diphosphonsäure oder ein Salz davon.

**18.** 3-[N-(3-(4-Methoxyphenoxy)propylamino]-1-hydroxy-propan-1,1-diphosphonsäure oder ein Salz davon.

**19.** 3-[N-(4-(Phenylthiobutyl)-N-methyl-amino]-1-hydroxy-propan-1,1-diphosphonsäure oder ein Salz davon.

**20.** 3-{N-[2-(3-Trifluomiethylphenylamino)äthyl]-N-methyl-amino}-1-hydroxy-propan-1,1-diphosphonsäure oder ein Salz davon.

**21.** 3-{N-[3-(2-Pyridylamino)propyl]-N-methyl-amino}-1-hydroxy-propan-1,1-diphosphonsäure oder ein Salz davon.

**22.** 3-{N-[3-(2-Pyrimidinyl amino)propyl]-N-methyl-amino}-1-hydroxy-propan-1,1-diphosphonsäure oder ein Salz davon.

**23.** 3-[N-(2-(Benzolsulfonyl)äthyl]-N-methyl-amino]-1-hydroxy-propan-1,1-diphosphonsäure oder ein Salz davon.

**24.** 3-{N-[3-(4-Methoxyphenoxy)propyl]-N-methyl-amino}-1-hydroxy-propan-1,1-diphosphonsäure oder ein Salz davon.

**25.** 3-{N-[3-(3-Methylphenoxy)propyl]-N-methyl-amino}-1-hydroxy-propan-1,1-diphosphonsäure oder ein Salz davon.

**26.** 3-{N-[3-(4-Fluorphenoxy)propyl]-N-methyl-amino}-1-hydroxy-propan-1,1-diphosphonsäure oder ein Salz davon.

**27.** 3-[N-(4-(Phenoxybutyl)-N-methyl-amino]-1-hydroxy-propan-1,1-diphosphonsäure oder ein Salz davon.

**28.** 3-[N-(6-(Phenoxyhexyl)-N-methyl-amino]-1-hydroxy-propan-1,1-diphosphonsäure oder ein Salz davon.

**29.** 3-{N-[2-(4-Chlorphenoxy)äthyl]-N-methyl-amino-1-hydroxy-propan-1,1-diphosphonsäure oder ein Salz davon.

**30.** 3-{{N-{3-[N'-(Pyrid-2-yl)-N'-äthyl-amino]propyl}-N-äthyl-amino}}-1-hydroxy-propan-1,1-diphosphonsäure oder ein Salz davon.

**31.** 3-{N-[2-(2-Hydroxphenoxy)äthyl]-N-methyl-amino}-1-hydroxy-propan-1,1-diphosphonsäure oder ein Salz davon.

**32.** Verbindungen gemäss einem der Ansprüche 1, 3 bis 5, 7, 9, 10 und 29 bis 31 zur Anwendung in einem Verfahren zur Behandlung des menschlichen oder tierischen Körpers.

**33.** Verbindungen gemäss einem der Ansprüche 2, 6, 8 und 11 bis 28 zur Anwendung in einem Verfahren zur Behandlung des menschlichen oder tierischen Körpers.

**34.** Pharmazeutische Präparate, enthaltend eine Verbindung gemäss einem der Ansprüche 1, 3 bis 5, 7, 9, 10 und 29 bis 31 und oder ein pharmazeutisch verwendbares Salz davon neben üblichen pharmazeutischen Hilfs- und Trägerstoffen.

**35.** Pharmazeutische Präparate, enthaltend eine Verbindung gemäss einem der Ansprüche 2, 6, 8 und 11 bis 28 oder ein pharmazeutisch verwendbares Salz davon neben üblichen pharmazeutischen Hilfs- und Trägerstoffen.

**36.** Verfahren zur Herstellung von Verbindungen der Formel I

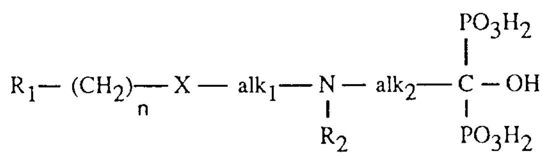

(I),

worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ Wasserstoff oder einen einwertigen aliphatischen Rest bedeutet, und ihrer Salze, dadurch gekennzeichnet, dass man

a) in einer Verbindung der Formel

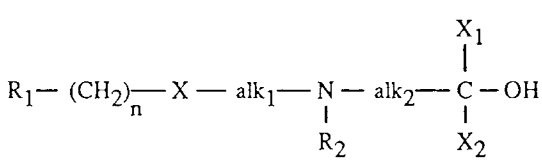

(II),

worin $R_1$, n, X, $alk_1$, $R_2$ und $alk_2$ die angegebenen Bedeutungen haben, $X_1$ eine funktionell abgewandelte und $X_2$ eine freie oder funktionell abgewandelte Phosphonogruppe bedeutet, funktionell abgewandeltes Phosphono $X_1$ und gegebenenfalls $X_2$ in die freie Phosphonogruppe überführt oder

b) Verbindungen der Formeln

$$R_1-(CH_2)_n-X-alk_1-Y_1 \quad (III)$$

und

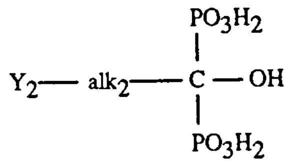

(IV),

worin einer der Reste $Y_1$ und $Y_2$ eine reaktionsfähige veresterte Hydroxygruppe Y und der andere eine Gruppe der Formel $-N(R_2)-H$ bedeutet, oder deren Salze oder unter reduzierenden Bedingungen Verbindungen der Formeln

$$R_1-(CH_2)_n-X-alk'_1{=}O \quad \text{(IIIa)}$$

und

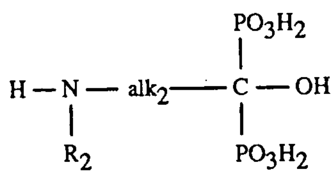

(IVa),

worin $alk_1'$ einen dem Rest $alk_1$ entsprechenden doppelt gebundenen Rest, z.B. einen entsprechend substituierten Niederalkanylylidenrest, darstellt, miteinander umsetzt oder

c) eine Verbindung der Formel

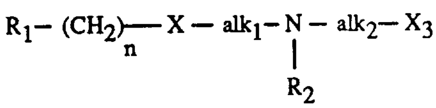

(V),

worin $X_3$ Carboxy, Carbamyl oder Cyano bedeutet, mit einem Phosphorylierungsmittel umsetzt, das Primärprodukt hydrolysiert und in einem ausgehend von Verbindungen der Formel V, worin $X_3$ Cyano oder Carbamyl ist, erhaltenen Zwischenprodukt der Formel

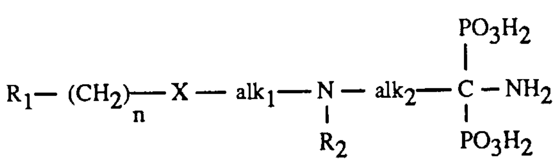

(VI),

bzw. einem Salz davon die Aminogruppe durch Behandlung mit salpetriger Säure durch Hydroxy ersetzt oder

d) Verbindungen der Formeln

$$R_1-(CH_2)_n-Y_3 \quad \text{(VII)}$$

und

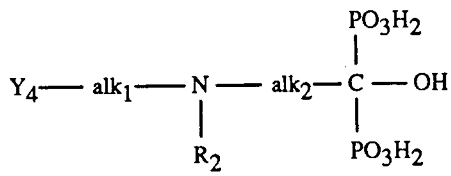 (VIII),

worin $Y_3$ eine Gruppe der Formel $-X_0-H$ und $Y_4$ reaktionsfähiges verestertes Hydroxy Y bedeutet oder, sofern n von 0 verschieden ist, $Y_3$ eine Gruppe Y und $Y_4$ eine Gruppe $-X_0-H$ darstellt, wobei $X_0$ Oxy, Thio oder gegebenenfalls aliphatisch substituiertes Imino X oder intermediär geschütztes Imino $-NR_0-$, worin $R_0$ eine übliche Aminoschutzgruppe ist, bedeutet und Y reaktionsfähiges verestertes Hydroxy darstellt, oder deren Salze oder zur Herstellung von Verbindungen der Formel I, worin X eine gegebenenfalls aliphatisch substituierte Iminogruppe darstellt, unter reduzierenden Bedingungen Verbindungen der Formeln

$$R_1-(CH_2)_{n-1}-CH=O \quad (VIIa)$$

und

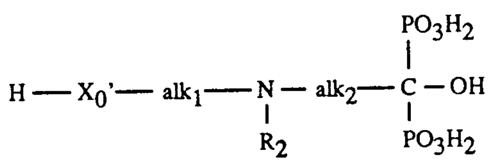 (VIIIa),

bzw.

$$R_1-(CH_2)_n-X_0'-H \qquad (VIIb)$$

und

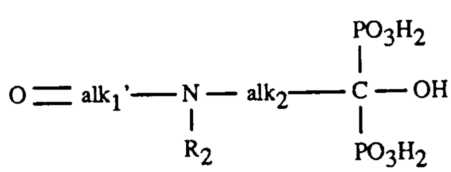 (VIIIb),

worin $R_1$, n, $alk_1$, $R_2$ und $alk_2$ die angegebenen Bedeutungen haben und $X'_0$ gegebenenfalls aliphatisch substituiertes Imino X oder intermediär geschütztes Imino $-NR_0-$ darstellt, wobei $R_0$ eine übliche Aminoschutzgruppe ist, miteinander umsetzt, jeweils die Aminoschutzgruppe $R_0$, sofern vorhanden abspaltet und gewünschtenfalls zur Herstellung von Verbindungen der Formel I, worin $R_2$ einen einwertigen aliphatischen Rest bedeutet, und ihrer Salze

e) Verbindungen der Formeln

$R_2-Y$ (IX)

und

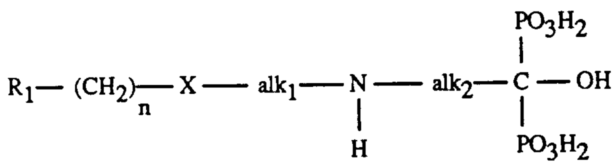 (Ia),

worin $R_1$, n, X, $alk_1$, $R_2$ und $alk_2$ die angegebenen Bedeutungen haben und Y für reaktionsfähiges verestertes Hydroxy bedeutet, oder ihre Salze oder unter reduzierenden Bedingungen Verbindungen der Formeln

$R_2'' = O$ (IXa)

und

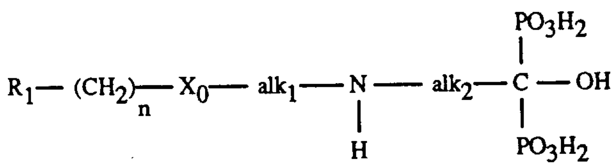 (Ia),

worin $R_1$, n, X, $alk_1$ und $alk_2$ die angegebenen Bedeutungen haben, $X_0$ Oxy, Thio oder gegebenenfalls aliphatisch substituiertes Imino X oder intermediär geschütztes Imino $-NR_0-$, worin $R_0$ eine übliche Aminoschutzgruppe ist, und $R_2''$ für einen dem Rest $R_2$ entsprechenden doppeltgebundenen Rest, z.B. für Niederalkyliden, steht, miteinander umsetzt, oder in anderer Weise eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**Patentansprüche für folgende Vertragsstaaten : ES, GR**

**1.** Verfahren zur Herstellung von Verbindungen der Formel I

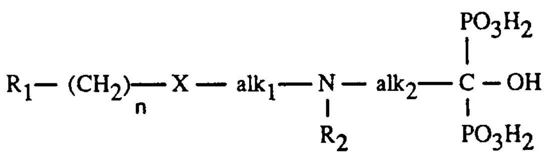 (I),

worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ Wasserstoff oder einen einwertigen aliphatischen Rest bedeutet, und ihrer Salze, dadurch gekennzeichnet, dass man

a) in einer Verbindung der Formel

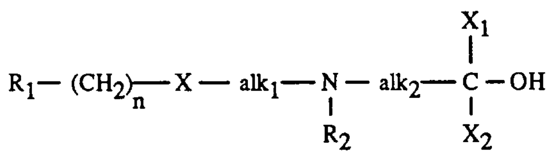

(II),

worin $R_1$, n, X, $alk_1$, $R_2$ und $alk_2$ die angegebenen Bedeutungen haben, $X_1$ eine funktionell abgewandelte und $X_2$ eine freie oder funktionell abgewandelte Phosphonogruppe bedeutet, funktionell abgewandeltes Phosphono $X_1$ und gegebenenfalls $X_2$ in die freie Phosphonogruppe überführt oder

b) Verbindungen der Formeln

$$R_1-(CH_2)_n-X-alk_1-Y_1 \quad \text{(III)}$$

und

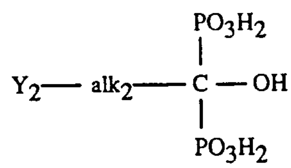

(IV),

worin einer der Reste $Y_1$ und $Y_2$ eine reaktionsfähige veresterte Hydroxygruppe Y und der andere eine Gruppe der Formel $-N(R_2)-H$ bedeutet, oder deren Salze oder unter reduzierenden Bedingungen Verbindungen der Formeln

$$R_1-(CH_2)_n-X-alk'_1=O \quad \text{(IIIa)}$$

und

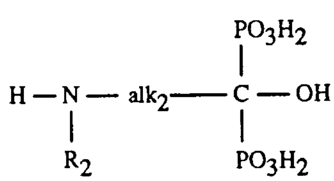

(IVa),

worin $alk_1$' einen dem Rest $alk_1$ entsprechenden doppelt gebundenen Rest, z.B. einen entsprechend substituierten Niederalkanylylidenrest, darstellt, miteinander umsetzt oder

c) eine Verbindung der Formel

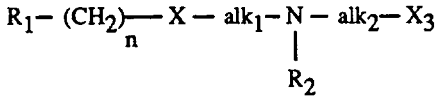

(V),

worin $X_3$ Carboxy, Carbamyl oder Cyano bedeutet, mit einem Phosphorylierungsmittel umsetzt, das Primärprodukt hydrolysiert und in einem ausgehend von Verbindungen der Formel V, worin $X_3$ Cyano oder Carbamyl ist, erhaltenen Zwischenprodukt der Formel

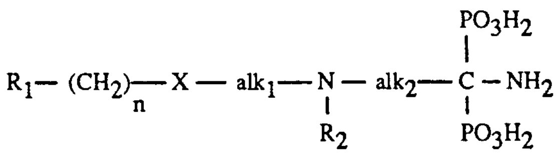

(VI),

bzw. einem Salz davon die Aminogruppe durch Behandlung mit salpetriger Säure durch Hydroxy ersetzt oder

d) Verbindungen der Formeln

$$R_1-(CH_2)_n-Y_3 \quad \text{(VII)}$$

und

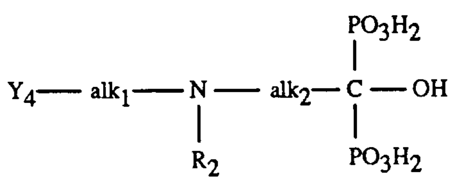

(VIII),

worin $Y_3$ eine Gruppe der Formel $-X_0-H$ und $Y_4$ reaktionsfähiges verestertes Hydroxy Y bedeutet oder, sofern n von 0 verschieden ist, $Y_3$ eine Gruppe Y und $Y_4$ eine Gruppe $-X_0-H$ darstellt, wobei $X_0$ Oxy, Thio oder gegebenenfalls aliphatisch substituiertes Imino X oder intermediär geschütztes Imino $-NR_0-$, worin $R_0$ eine übliche Aminoschutzgruppe ist, bedeutet und Y reaktionsfähiges verestertes Hydroxy darstellt, oder deren Salze oder zur Herstellung von Verbindungen der Formel I, worin X eine gegebenenfalls aliphatisch substituierte Iminogruppe darstellt, unter reduzierenden Bedingungen Verbindungen der Formeln

$$R_1-(CH_2)_{n-1}-CH=O \quad \text{(VIIa)}$$

und

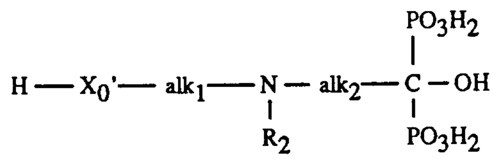 (VIIIa),

bzw.

$R_1-(CH_2)_n-X_0'-H$ (VIIb)

und

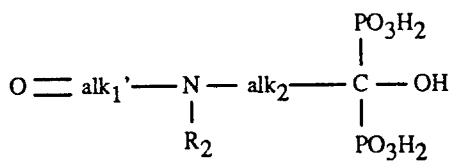 (VIIIb),

worin $R_1$, n, $alk_1$, $R_2$ und $alk_2$ die angegebenen Bedeutungen haben und $X'_0$ gegebenenfalls aliphatisch substituiertes Imino X oder intermediär geschütztes Imino $-NR_0-$ darstellt, wobei $R_0$ eine übliche Aminoschutzgruppe ist, miteinander umsetzt, jeweils die Aminoschutzgruppe $R_0$, sofern vorhanden abspaltet und gewünschtenfalls zur Herstellung von Verbindungen der Formel I, worin $R_2$ einen einwertigen aliphatischen Rest bedeutet, und ihrer Salze

e) Verbindungen der Formeln

$R_2$-Y (IX)

und

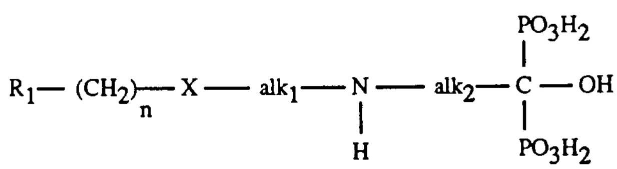 (Ia),

worin $R_1$, n, X, $alk_1$, $R_2$ und $alk_2$ die angegebenen Bedeutungen haben und Y für reaktionsfähiges verestertes Hydroxy bedeutet, oder ihre Salze oder unter reduzierenden Bedingungen Verbindungen der Formeln

$R_2''=O$ (IXa)

und

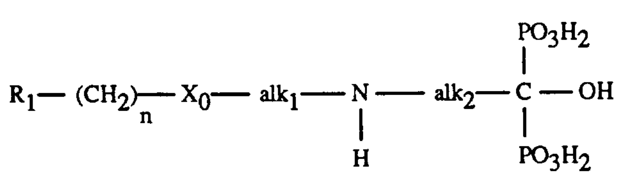 (Ia),

worin $R_1$, n, X, $alk_1$ und $alk_2$ die angegebenen Bedeutungen haben, $X_0$ Oxy, Thio oder gegebenenfalls aliphatisch substituiertes Imino X oder intermediär geschütztes Imino $-NR_0-$, worin $R_0$ eine übliche Aminoschutzgruppe ist, und $R_2''$ für einen dem Rest $R_2$ entsprechenden doppeltgebundenen Rest, z.B. für Niederalkyliden, steht, miteinander umsetzt, oder in anderer Weise eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**2.** Verfahren gemäss Anspruch 1 zur Herstellung von Verbindungen der Formel I, worin $R_1$ einen aromatischen Rest bedeutet, n 0, 1, 2 oder 3 darstellt, X für eine Oxy-, gegebenenfalls oxydierte Thio- oder gegebenenfalls aliphatisch substituierte Iminogruppe steht, $alk_1$ und $alk_2$ gleiche oder verschiedene zweiwertige aliphatische Reste darstellen und $R_2$ einen einwertigen aliphatischen Rest bedeutet, und ihrer Salze.

**3.** Verfahren gemäss Anspruch 1 zur Herstellung von Verbindungen der Formel I, worin $R_1$ einen unsubstituierten oder durch Niederalkyl, Niederalkoxy, Niederalkylendioxy, Hydroxy, Halogen und/oder Trifluormethyl mono-, di- oder trisubstituierten Phenyl-, Naphthyl-, Imidazolyl-, Thiazolyl- Oxazolyl-, Pyridyl-, Pyrimidinyl- oder Pyridazinylrest bedeutet, n für 0, 1, 2 oder 3 steht, X Oxy, Thio, Sulfinyl, Sulfonyl, Imino oder Niederalkylimino darstellt, $alk_1$ Niederalkylen bedeutet, $R_2$ für Wasserstoff, Niederalkyl oder Niederalkenyl steht und $alk_2$ Niederalkylen bedeutet, und ihrer Salze, insbesondere ihre pharmazeutisch verwendbaren Salze.

**4.** Verfahren gemäss Anspruch 1 zur Herstellung von Verbindungen der Formel I, worin $R_1$ einen unsubstituierten oder durch $C_1$-$C_4$-Alkyl, $C_1$-$C_4$-Alkoxy, $C_1$-$C_4$-Alkylendioxy, Hydroxy, Halogen der Atomnummer bis und mit 35 und/oder Trifluormethyl mono- oder disubstituierten Phenylrest bedeutet, n Für 0, 1 oder 2 steht, X Oxy, Thio, Sulfonyl, Imino oder $C_1$-$C_4$-Alkylimino darstellt, $alk_1$ $C_2$-$C_6$-Alkylen bedeutet, $R_2$ für Wasserstoff oder $C_1$-$C_4$-Alkyl steht und $alk_2$ $C_2$-$C_4$-Alkylen darstellt, und ihre Salze.

**5.** Verfahren gemäss Anspruch 1 zur Herstellung von Verbindungen der Formel I, worin $R_1$ einen unsubstituierten oder durch $C_1$-$C_4$-Alkyl und/oder unsubstituiertes oder durch $C_1$-$C_4$-Alkyl, $C_1$-$C_4$-Alkoxy, $C_1$-$C_4$-Alkylendioxy, Hydroxy, Halogen der Atomnummer bis und mit 35 und/oder Trifluormethyl mono- oder disubstituiertes Phenyl substituierten Pyridyl- oder Pyrimidinylrest bedeutet, n für 0, 1 oder 2 steht, X Oxy, Thio, Imino oder $C_1$-$C_4$-Alkylimino darstellt, $alk_1$ $C_2$-$C_6$-Alkylen bedeutet, $R_2$ für Wasserstoff oder $C_1$-$C_4$-Alkyl steht und $alk_2$ $C_2$-$C_4$-Alkylen darstellt, und ihrer Salze.

**6.** Verfahren gemäss Anspruch 2 zur Herstellung von Verbindungen der Formel I, worin $R_1$ einen unsubstituierten oder durch $C_1$-$C_4$-Alkyl, $C_1$-$C_4$-Alkoxy, $C_1$-$C_4$-Alkylendioxy, Hydroxy, Halogen der Atomnummer bis und mit 35 und/oder Trifluormethyl mono- oder disubstituierten Phenyl-, Naphthyl-, Pyridyl- oder Pyrimidinylrest bedeutet, n für 0, 1 oder 2 steht, X Oxy, Thio, Sulfonyl, Imino oder $C_1$-$C_4$-Alkylimino darstellt, $alk_1$ $C_2$-$C_6$-Alkylen bedeutet, $R_2$ für $C_1$-$C_4$-Alkyl steht und $alk_2$ $C_2$-$C_4$-Alkylen darstellt, und ihrer Salze.

**7.** Verfahren gemäss Anspruch 1 zur Herstellung von Verbindungen der Formel I, worin $R_1$ unsubstituiertes oder durch $C_1$-$C_4$-Alkyl, $C_1$-$C_4$-Alkoxy, Hydroxy, Halogen der Atomnummer bis und mit 35 oder Trifluormethyl substituiertes Phenyl oder unsubstituiertes Pyridyl oder Pyrimidinyl bedeutet, n für 0 steht, X Oxy, Thio, Sulfonyl, Imino oder Methylimino bedeutet, $alk_1$ $C_2$-$C_6$-Alkylen darstellt, $R_2$ Wasserstoff oder $C_1$-$C_4$-Alkyl bedeutet und $alk_2$ Aethylen ist, und ihrer Salze.

**8.** Verfahren gemäss Anspruch 2 zur Herstellung von Verbindungen der Formel I, worin $R_1$ unsubstituiertes oder durch $C_1$-$C_4$-Alkyl, $C_1$-$C_4$-Alkoxy, Hydroxy, Halogen der Atomnummer bis und mit 35 oder Trifluormethyl substituiertes Phenyl oder unsubstituiertes Pyridyl oder Pyrimidinyl bedeutet, n für 0 steht, X Oxy, Thio, Sulfonyl, Imino oder Methylimino bedeutet, $alk_1$ $C_2$-$C_6$-Alkylen darstellt, $R_2$ $C_1$-$C_3$-Alkyl bedeutet und $alk_2$ Aethylen ist, und ihrer Salze.

**9.** Verfahren gemäss Anspruch 1 zur Herstellung von Verbindungen der Formel I, worin $R_1$ unsubstituiertes oder durch $C_1$-$C_4$-Alkyl, $C_1$-$C_4$-Alkoxy oder Halogen der Atomnummer bis und mit 35 substituiertes Phenyl bedeutet, n für 0 steht, X Oxy oder Thio bedeutet, $alk_1$ $C_2$-$C_4$-Alkylen darstellt, $R_2$ $C_1$-$C_3$-Alkyl

oder Wasserstoff bedeutet und $alk_2$ Aethylen ist, und ihrer Salze.

**10.** Verfahren gemäss Anspruch 1 zur Herstellung von Verbindungen der Formel I, worin $R_1$ unsubstituiertes oder durch $C_1$-$C_4$-Alkyl oder Phenyl substituiertes Pyridyl oder Pyrimidinyl bedeutet, n für 0 steht, X unsubstituiertes oder durch $C_1$-$C_4$-Alkyl substituiertes, Imino, Oxy oder Thio bedeutet, $alk_1$ $C_2$-$C_4$-Alkylen bedeutet und $R_2$ $C_1$-$C_3$-Alkyl oder Wasserstoff bedeutet und $alk_2$ Aethylen ist, und ihrer Salze.

**11.** Verfahren gemäss Anspruch 1 oder 2 zur Herstellung von 3-[N-(3-Phenoxypropyl)-N-methyl-amino]-1-hydroxy-propan-1,1-diphosphonsäure oder eines Salzes davon.

**12.** Verfahren gemäss Anspruch 1 zur Herstellung von 3-{N-[2-(4-Methoxyphenoxy)äthyl]amino}-1-hydroxy-propan-1,1-diphosphonsäure oder eines Salzes davon.

**13.** Verfahren gemäss Anspruch 1 oder 2 zur Herstellung von 3-[N-(2-Phenoxyäthyl)-N-methyl-amino]-1-hydroxy-propan-1,1-diphosphonsäure oder eines Salzes davon.

**14.** Verfahren gemäss Anspruch 1 oder 2 zur Herstellung von 3-[N-(2-Phenylthioäthyl)-N-methyl-amino]-1-hydroxy-propan-1,1-diphosphonsäure oder eines Salzes davon.

**15.** Verfahren gemäss Anspruch 1 zur Herstellung von 3-[N-(3-Phenoxypropyl)amino]-1-hydroxy-propan-1,1-diphosphonsäure oder eines Salzes davon.

**16.** Verfahren gemäss Anspruch 1 oder 2 zur Herstellung von 3-[N-(3-Phenylthiopropyl)-N-methyl-amino]-1-hydroxy-propan-1,1-diphosphonsäure oder eines Salzes davon.

**17.** Verfahren gemäss Anspruch 1 oder 2 zur Herstellung von 3-{N-[3-(4-Chlorphenoxy)propyl]-N-methyl-amino-1-hydroxy-propan-1,1-diphosphonsäure oder eines Salzes davon.

**18.** Verfahren gemäss Anspruch 1 zur Herstellung von 3-[N-(3-(4-Methoxyphenoxy)propylamino]-1-hydroxy-propan-1,1-diphosphonsäure oder eines Salzes davon.

**19.** Verfahren gemäss Anspruch 1 oder 2 zur Herstellung von 3-[N-(4-(Phenylthiobutyl)-N-methyl-amino]-1-hydroxy-propan-1,1-diphosphonsäure oder eines Salzes davon.

**20.** Verfahren gemäss Anspruch 1 oder 2 zur Herstellung von 3-{N-[2-(3-Trifluormethylphenylamino)äthyl]-N-methyl-amino}-1-hydroxy-propan-1,1-diphosphonsäure oder eines Salzes davon.

**21.** Verfahren gemäss Anspruch 1 oder 2 zur Herstellung von 3-{N-[3-(2-Pyridylamino)propyl]-N-methyl-amino}-1-hydroxy-propan-1,1-diphosphonsäure oder eines Salzes davon.

**22.** Verfahren gemäss Anspruch 1 oder 2 zur Herstellung von 3-{N-[3-(2-Pyrimidinyamino)propyl]-N-methyl-amino}-1-hydroxy-propan-1,1-diphosphonsäure oder eines Salzes davon.

**23.** Verfahren gemäss Anspruch 1 oder 2 zur Herstellung von 3-[N-(2-(Benzolsulfonyl)äthyl]-N-methyl-amino]-1-hydroxy-propan-1,1-diphosphonsäure oder eines Salzes davon.

**24.** Verfahren gemäss Anspruch 1 oder 2 zur Herstellung von 3-{N-[3-(4-Methoxyphenoxy)propyl]-N-methyl-amino}-1-hydroxy-propan-1,1-diphosphonsäure oder eines Salzes davon.

**25.** Verfahren gemäss Anspruch 1 oder 2 zur Herstellung von 3-{N-[3-(3-Methylphenoxy)propyl]-N-methyl-amino}-1-hydroxy-propan-1,1-diphosphonsäureoder eines Salzes davon.

**26.** Verfahren gemäss Anspruch 1 oder 2 zur Herstellung von 3-{N-[3-(4-Fluorphenoxy)propyl]-N-methyl-amino}-1-hydroxy-propan-1,1diphosphonsäure oder eines Salzes davon.

**27.** Verfahren gemäss Anspruch 1 oder 2 zur Herstellung von 3-[N-(4-(Phenoxybutyl)-N-methyl-amino]-1-hydroxy-propan-1,1-diphosphonsäure oder eines Salzes davon.

**28.** Verfahren gemäss Anspruch 1 oder 2 zur Herstellung von 3-[N-(6-(Phenoxyhexyl)-N-methyl-amino]-1-hydroxy-propan-1,1-diphosphonsäure oder eines Salzes davon.

**29.** Verfahren gemäss Anspruch 1 oder 2 zur Herstellung von 3-{N-[2-(4-Chlorphenoxy)äthyl]-N-methyl-amino-1-hydroxy-propan-1,1-diphosphonsäure oder eines Salzes davon.

**30.** Verfahren gemäss Anspruch 1 oder 2 zur Herstellung von 3-{{N-{3-[N'-(Pyrid-2-yl)-N'-äthyl-amino]-propyl}-N-äthyl-amino}}-1-hydroxy-propan-1,1-diphosphonsäure oder eines Salzes davon.

**31.** Verfahren gemäss Anspruch 1 oder 2 zur Herstellung von 3-{N-[2-(2-Hydroxyphenoxy)äthyl]-N-methyl-amino}-1-hydroxy-propan-1,1-diphosphonsäure oder eines Salzes davon.

**32.** Verfahren zur Herstellung pharmazeutischer Präparate, dadurch gekennzeichnet, dass man eine Verbindung, erhältlich gemäss einem der Ansprüche 1 bis 32 oder ein pharmazeutisch verwendbares Salz davon mit üblichen pharmazeutischen Hilfs- und Trägerstoffen vermischt.

**Claims**
**Claims for the following Contracting States : AT, BE, CH, DE, DK, FR, GB, IT, LI, LU, NL, SE**

**1.** A compound of the formula I

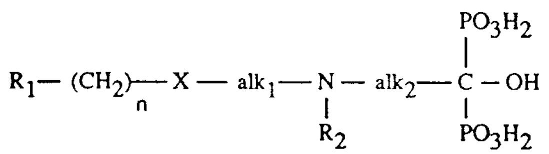

(I)

in which $R_1$ is an aromatic radical, n is 0, 1, 2 or 3, X is an oxy group, a thio group which can be oxidized or an imino group which is unsubstituted or has aliphatic substituents, $alk_1$ and $alk_2$ are identical or different divalent aliphatic radicals, and $R_2$ is hydrogen or a monovalent aliphatic radical, and a salt thereof.

**2.** A compound according to claim 1 of the formula I in which $R_1$ is an aromatic radical, n is 0, 1, 2 or 3, X is an oxy group, a thio group which can be oxidized or an imino group which is unsubstituted or has aliphatic substituents, $alk_1$ and $alk_2$ are identical or different divalent aliphatic radicals, and $R_2$ is a monovalent aliphatic radical, and a salt thereof.

**3.** A compound according to claim 1 of the formula I in which $R_1$ is a phenyl, naphthyl, imidazolyl, thiazolyl, oxazolyl, pyridyl, pyrimidinyl or pyridazinyl radical which is unsubstituted or monosubstituted, disubstituted or trisubstituted by lower alkyl, lower alkoxy, lower alkylenedioxy hydroxyl, halogen and/or trifluoromethyl, n is 0, 1, 2 or 3, X is oxy, thio, sulfinyl, sulfonyl, imino or lower alkylimino, $alk_1$ is lower alkylene, $R_2$ is hydrogen, lower alkyl or lower alkenyl and $alk_2$ is lower alkylene, and a salt thereof, in particular a pharmaceutically acceptable salt thereof.

**4.** A compound according to claim 1 of the formula I in which $R_1$ is a phenyl radical which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, sulfonyl, imino or $C_1$-$C_4$alkylimino, $alk_1$ is $C_2$-$C_6$alkylene, $R_2$ is hydrogen or $C_1$-$C_4$alkyl and $alk_2$ is $C_2$-$C_4$alkylene, and a salt thereof.

**5.** A compound according to claim 1 of the formula I in which $R_1$ is a pyridyl or pyrimidinyl radical which is unsubstituted or substituted by $C_1$-$C_4$alkyl and/or phenyl which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, imino or $C_1$-$C_4$alkylimino, $alk_1$ is $C_2$-$C_6$alkylene, $R_2$ is hydrogen or $C_1$-$C_4$alkyl and $alk_2$ is $C_2$-$C_4$alkylene, and a salt thereof.

6. A compound according to claim 2 of the formula I in which $R_1$ is a phenyl, naphthyl, pyridyl or pyrimidinyl radical which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, sulfonyl, imino or $C_1$-$C_4$alkylimino, $alk_1$ is $C_2$-$C_6$alkylene, $R_2$ is $C_1$-$C_4$alkyl and $alk_2$ is $C_2$-$C_4$alkylene, and a salt thereof.

7. A compound according to claim 1 of the formula I in which $R_1$ is phenyl which is unsubstituted or substituted by $C_1$-$C_4$alkyl, $C_1$-$C_4$alkoxy, hydroxyl, halogen having an atomic number up to and including 35 or trifluoromethyl, or $R_1$ is unsubstituted pyridyl or pyrimidinyl, n is 0, X is oxy, thio, sulfonyl, imino or methylimino, $alk_1$ is $C_2$-$C_6$alkylene, $R_2$ is hydrogen or $C_1$-$C_4$alkyl and $alk_2$ is ethylene, and a salt thereof.

8. A compound according to claim 2 of the formula I in which $R_1$ is phenyl which is unsubstituted or substituted by $C_1$-$C_4$alkyl, $C_1$-$C_4$alkoxy, hydroxyl, halogen having an atomic number up to and including 35 or trifluoromethyl, or $R_1$ is unsubstituted pyridyl or pyrimidinyl, n is 0, X is oxy, thio, sulfonyl, imino or methylimino, $alk_1$ is $C_2$-$C_6$alkylene, $R_2$ is $C_1$-$C_3$alkyl and $alk_2$ is ethylene, and a salt thereof.

9. A compound according to claim 1 of the formula I in which $R_1$ is phenyl which is unsubstituted or substituted by $C_1$-$C_4$alkyl, $C_1$-$C_4$alkoxy or halogen having an atomic number up to and including 35, n is 0, X is oxy or thio, $alk_1$ is $C_2$-$C_4$alkylene, $R_2$ is $C_1$-$C_3$alkyl or hydrogen and $alk_2$ is ethylene, and a salt thereof.

10. A compound according to claim 1 of the formula I in which $R_1$ is pyridyl or pyrimidinyl which is unsubstituted or substituted by $C_1$-$C_4$alkyl or phenyl, n is 0, X is imino which is unsubstituted or substituted by $C_1$-$C_4$alkyl, or is oxy or thio, $alk_1$ is $C_2$-$C_4$alkylene, $R_2$ is $C_1$-$C_3$alkyl or hydrogen and $alk_2$ is ethylene, and a salt thereof.

11. 3-[N-(3-Phenoxypropyl)-N-methylamino]-1-hydroxypropane-1,1-diphosphonic acid or a salt thereof.

12. 3-{N-[2-(4-Methoxyphenoxy)-ethyl]-amino}-1-hydroxypropane-1,1-diphosphonic acid or a salt thereof.

13. 3-[N-(2-Phenoxyethyl)-N-methylamino]-1-hydroxypropane-1,1-diphosphonic acid or a salt thereof.

14. 3-[N-(2-Phenylthioethyl)-N-methylamino]-1-hydroxypropane-1,1-diphosphonic acid or a salt thereof.

15. 3-[N-(3-Phenoxypropyl)amino]-1-hydroxypropane-1,1-diphosphonic acid or a salt thereof.

16. 3-[N-(3-Phenylthiopropyl)-N-methylamino]-1-hydroxypropane-1,1-diphosphonic acid or a salt thereof.

17. 3-{N-[3-(4-Chlorophenoxy)propyl]-N-methylamino}-1-hydroxypropane-1,1-diphosphonic acid or a salt thereof.

18. 3-[N-(3-(4-Methoxyphenoxy)propyl)amino]-1-hydroxypropane-1,1-diphosphonic acid or a salt thereof.

19. 3-[N-(4-(Phenylthiobutyl)-N-methyl)amino]-1-hydroxypropane-1,1-diphosphonic acid or a salt thereof.

20. 3-{N-[2-(3-Trifluoromethylphenylamino)-ethyl]-N-methylamino)-1-hydroxypropane-1,1-diphosphonic acid or a salt thereof.

21. 3-{N-[3-(2-Pyrimidylamino)-propyl]-N-methylamino}-1-hydroxypropane-1,1-diphosphonic acid or a salt thereof.

22. 3-{N-[3-(2-Pyrimidinylamino)-propyl]-N-methylamino)-1-hydroxypropane-1,1-diphosphonic acid or a salt thereof.

23. 3-[N-(2-(Benzosulfonyl)-ethyl)-N-methylamino]-1-hydroxypropane-1,1-diphosphonic acid or a salt thereof.

**24.** 3-{N-[3-(4-Methoxyphenoxy)-propyl]-N-methylamino}-1-hydroxypropane-1,1-diphosphonic acid or a salt thereof.

**25.** 3-{N-[3-(3-Methylphenoxy)-propyl]-N-methylamino}-1-hydroxypropane-1,1-diphosphonic acid or a salt thereof.

**26.** 3-{N-[3-(4-Fluorophenoxy)-propyl]-N-methylamino}-1-hydroxypropane-1,1-diphosphonic acid or a salt thereof.

**27.** 3-[N-(4-(Phenoxybutyl)-N-methyl)amino]-1-hydroxypropane-1,1-diphosphonic acid or a salt thereof.

**28.** 3-[N-(6-(Phenoxyhexyl)-N-methyl)amino]-1-hydroxypropane-1,1-diphosphonic acid or a salt thereof.

**29.** 3-{N-[2-(4-Chlorophenoxy)-ethyl]-N-methylamino}-1-hydroxypropane-1,1-diphosphonic acid or a salt thereof.

**30.** 3-{{N-[3-[N'-(Pyrid-2-yl)-N'-ethylamino]propyl)-N-ethylamino}}-1-hydroxypropane-1,1-diphosphonic acid or a salt thereof.

**31.** 3-{N-[2-(2-Hydroxyphenoxy)-ethyl]-N-methylamino}-1-hydroxypropane-1,1-diphosphonic acid or a salt thereof.

**32.** A compound according to any one of claims 1, 3 to 5, 7, 9, 10 and 29 to 31 for use in a process for the treatment of the human or animal body.

**33.** A compound according to any one of claims 2, 6, 8 and 11 to 28 for use in a process for the treatment of the human or animal body.

**34.** A pharmaceutical formulation containing a compound according to any one of claims 1, 3 to 5, 7, 9, 10 and 29 to 31 and/or a pharmaceutically acceptable salt thereof together with customary pharmaceutical adjuncts and excipients.

**35.** A pharmaceutical formulation containing a compound according to any one of claims 2, 6, 8 and 11 to 28 or a pharmaceutically acceptable salt thereof together with customary pharmaceutical adjuncts and excipients.

**36.** A process for the preparation of a compound of the formula I

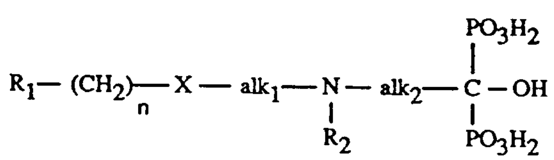

(I)

in which $R_1$ is an aromatic radical, n is 0, 1, 2 or 3, X is an oxy group, a thio group which can be oxidized or an imino group which is unsubstituted or has aliphatic substituents, $alk_1$ and $alk_2$ are identical or different divalent aliphatic radicals, and $R_2$ is hydrogen or a monovalent aliphatic radical, and a salt thereof, which comprises

a) in a compound of the formula

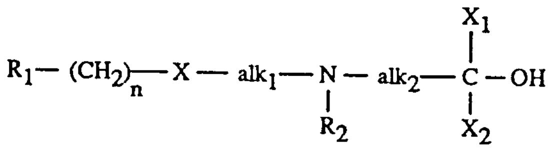

(II),

in which $R_1$, n, X, $alk_1$, $R_2$ and $alk_2$ are as defined above, $X_1$ is a functionally modified phosphono group and $X_2$ is a free or functionally modified phosphono group, converting functionally modified phosphono $X_1$ and, if appropriate, $X_2$ into the free phosphono group or

b) reacting with one another compounds of the formulae

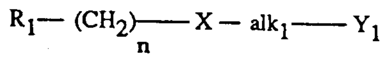

(III)

and

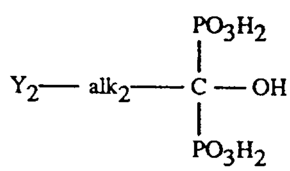

(IV),

in which one of the radicals $Y_1$ and $Y_2$ is a reactive esterified hydroxyl group Y and the other is a group of the formula $-N(R_2)-H$, or a salt thereof, or reacting with one another under reducing conditions compounds of the formulae

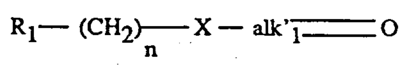

(IIIa)

and

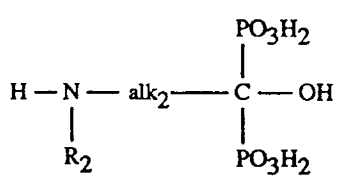

(IVa),

in which $alk'_1$ is a doubly bound radical corresponding to the radical $alk_1$, for example a correspondingly substituted lower alkanylylidene radical, or

c) reacting a compound of the formula

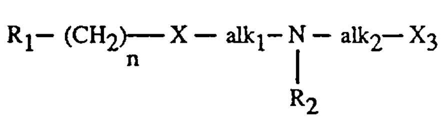

(V),

in which $X_3$ is carboxyl, carbamyl or cyano, with a phosphorylating agent, hydrolysing the primary product and, in an intermediate of the formula

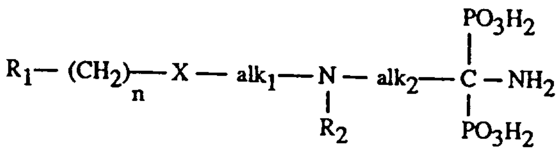

(VI),

or a salt thereof obtained using as starting materials compounds of the formula V in which $X_3$ is cyano or carbamyl, replacing the amino group by hydroxyl by treatment with nitrous acid or
d) reacting with one another compounds of the formulae

$$R_1-(CH_2)_n-Y_3 \qquad (VII)$$

and

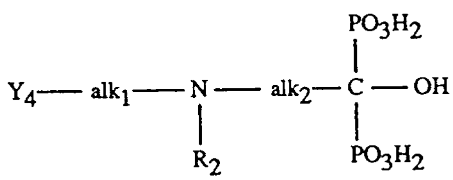

(VIII),

in which $Y_3$ is a group of the formula $-X_0$-H and $Y_4$ is reactive esterified hydroxyl Y or, if n is other than 0, $Y_3$ is a group Y and $Y_4$ is a group $-X_0$-H, $X_0$ being oxy, thio or imino X which is unsubstituted or has aliphatic substituents or is intermediately protected imino $-NR_0-$ in which $R_0$ is a customary amino protective group, and Y is reactive esterified hydroxyl, or a salt thereof, or, in order to prepare compounds of the formula I in which X is an imino group which is unsubstituted or has aliphatic substituents, reacting with one another, under reducing conditions, compounds of the formulae

$$R_1-(CH_2)_{n-1}-CH=O \quad (VIIa)$$

and

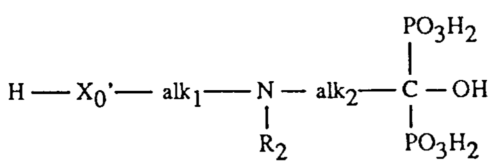

(VIIIa),

or

$$R_1-(CH_2)_n-X_0'-H \qquad (VIIb)$$

and

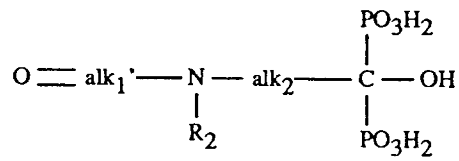 (VIIIb),

in which $R_1$, n, $alk_1$, $R_2$ and $alk_2$ are as defined above and $X'_0$ is imino X which is unsubstituted or has aliphatic substituents or is intermediately protected imino $-NR_0-$ in which $R_0$ is a customary amino protective group, and in each case splitting off the amino protective group $R_0$, if present, and, if desired in order to prepare compounds of the formula I in which $R_2$ is a monovalent aliphatic radical, and a salt thereof,

e) reacting with one another compounds of the formulae

$R_2$-Y (IX)

and

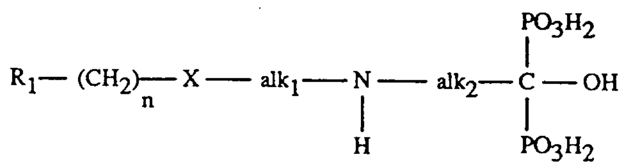 (Ia),

in which $R_1$, n, X, $alk_1$, $R_2$ and $alk_2$ are as defined above and Y is reactive esterified hydroxyl, or a salt thereof, or reacting with one another, under reducing conditions, compounds of the formulae

$R_2'' = O$ (IXa)

and

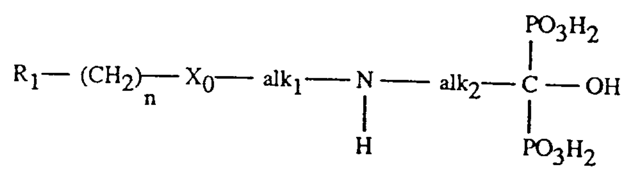 (Ia),

in which $R_1$, n, X, $alk_1$ and $alk_2$ are as defined above, $X_0$ is oxy, thio or imino X which is unsubstituted or has aliphatic substituents or is intermediately protected imino $-NR_0-$ in which $R_0$ is a customary amino protective group, and $R_2''$ is a doubly bound radical corresponding to the radical $R_2$, for example lower alkylidene, or converting a resulting compound into another compound of the formula I in another manner, separating a mixture of isomers obtainable in accordance with the process into its components and isolating the isomer preferred in the particular case and/or converting a free compound obtainable in accordance with the process into a salt or converting a salt obtainable in accordance with the process into the corresponding free compound.

**Claims for the following Contracting States : ES, GR**

**1.** A process for the preparation of a compound of the formula I

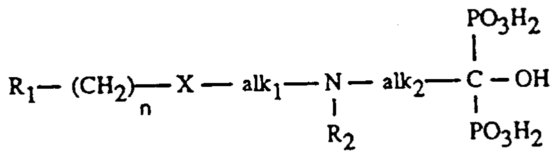

(I),

in which $R_1$ is an aromatic radical, n is 0, 1, 2 or 3, X is an oxy group, a thio group which can be oxidized or an imino group which is unsubstituted or has aliphatic substituents, $alk_1$ and $alk_2$ are identical or different divalent aliphatic radicals, and $R_2$ is hydrogen or a monovalent aliphatic radical, and a salt thereof, which comprises

a) in a compound of the formula

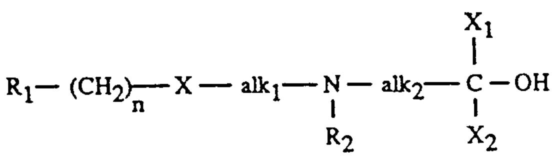

(II),

in which $R_1$, n, X, $alk_1$, $R_2$ and $alk_2$ are as defined above, $X_1$ is a functionally modified phosphono group and $X_2$ is a free or functionally modified phosphono group, converting functionally modified phosphono $X_1$ and, if appropriate, $X_2$ into the free phosphono group or

b) reacting with one another compounds of the formulae

$$R_1-(CH_2)_n-X-alk_1-Y_1 \quad (III)$$

and

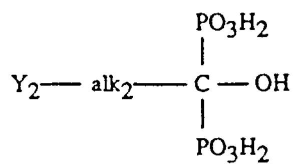

(IV),

in which one of the radicals $Y_1$ and $Y_2$ is a reactive esterified hydroxyl group Y and the other is a group of the formula $-N(R_2)-H$, or a salt thereof, or reacting with one another under reducing conditions compounds of the formulae

$$R_1-(CH_2)_n-X-alk'_1{=}O \quad (IIIa)$$

and

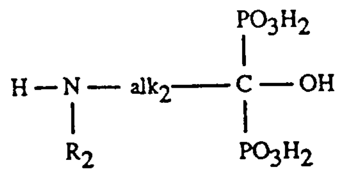 (IVa), ,

in which alk'$_1$ is a doubly bound radical corresponding to the radical alk$_1$, for example a correspondingly substituted lower alkanylylidene radical, or

c) reacting a compound of the formula

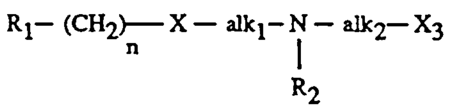 (V),

in which $X_3$ is carboxyl, carbamyl or cyano, with a phosphorylating agent, hydrolysing the primary product and, in an intermediate of the formula

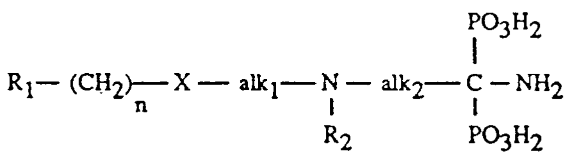 (VI),

or a salt thereof obtained using as starting materials compounds of the formula V in which $X_3$ is cyano or carbamyl, replacing the amino group by hydroxyl by treatment with nitrous acid or

d) reacting with one another compounds of the formulae

$$R_1-(CH_2)_n-Y_3 \quad (VII)$$

and

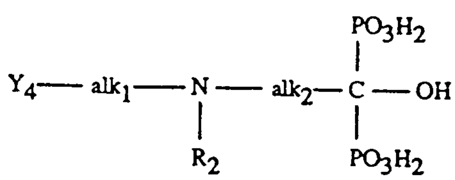 (VIII),

in which $Y_3$ is a group of the formula $-X_0-H$ and $Y_4$ is reactive esterified hydroxyl Y or, if n is other than 0, $Y_3$ is a group Y and $Y_4$ is a group $-X_0-H$, $X_0$ being oxy, thio or imino X which is unsubstituted or has aliphatic substituents or is intermediately protected imino $-NR_0-$ in which $R_0$ is a customary amino protective group, and Y is reactive esterified hydroxyl, or a salt thereof, or, in order to prepare compounds of the formula I in which X is an imino group which is unsubstituted or has aliphatic substituents, reacting with one another, under reducing conditions, compounds of the formulae

$$R_1-(CH_2)_{n-1}-CH{=}O \quad (VIIa)$$

and

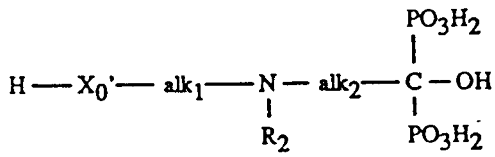
(VIIIa),

or

$R_1-(CH_2)_n-X_0'-H$ (VIIb)

and

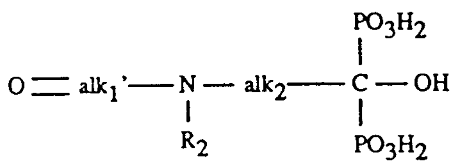
(VIIIb),

in which $R_1$, n, $alk_1$, $R_2$ and $alk_2$ are as defined above and $X'_0$ is imino X which is unsubstituted or has aliphatic substituents or is intermediately protected imino $-NR_0-$ in which $R_0$ is a customary amino protective group, and in each case splitting off the amino protective group $R_0$, if present, and, if desired in order to prepare compounds of the formula I in which $R_2$ is a monovalent aliphatic radical, and a salt thereof,
e) reacting with one another compounds of the formulae

$R_2$-Y (IX)

and

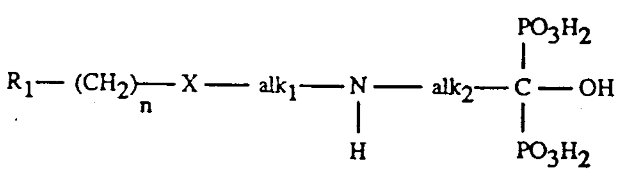
(Ia),

in which $R_1$, n, X, $alk_1$, $R_2$ and $alk_2$ are as defined above and Y is reactive esterified hydroxyl, or a salt thereof, or reacting with one another, under reducing conditions, compounds of the formulae

$R_2''=O$ (IXa)

and

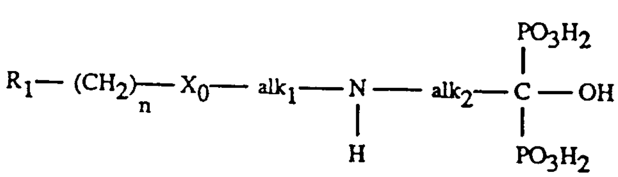
(Ia),

in which $R_1$, n, X, $alk_1$ and $alk_2$ are as defined above, $X_0$ is oxy, thio or imino X which is unsubstituted or has aliphatic substituents or is intermediately protected imino $-NR_0-$ in which $R_0$ is a customary amino protective group, and $R_2''$ is a doubly bound radical corresponding to the radical $R_2$, for example lower alkylidene, or converting a resulting compound into another compound of the formula I in another manner, separating a mixture of isomers obtainable in accordance with the process into its components and isolating the isomer preferred in the particular case and/or converting a free compound obtainable in accordance with the process into a salt or converting a salt obtainable in accordance with the process into the corresponding free compound.

**2.** A process according to claim 1 for the preparation of a compound of the formula I in which $R_1$ is an aromatic radical, n is 0, 1, 2 or 3, X is an oxy group, a thio group which can be oxidized or an imino group which is unsubstituted or has aliphatic substituents, $alk_1$ and $alk_2$ are identical or different divalent aliphatic radicals, and $R_2$ is a monovalent aliphatic radical, and a salt thereof.

**3.** A process according to claim 1 for the preparation of a compound of the formula I in which $R_1$ is a phenyl, naphthyl, imidazolyl, thiazolyl, oxazolyl, pyridyl, pyrimidinyl or pyridazinyl radical which is unsubstituted or monosubstituted, disubstituted or trisubstituted by lower alkyl, lower alkoxy, lower alkylenedioxy, hydroxyl, halogen and/or trifluoromethyl, n is 0, 1, 2 or 3, X is oxy, thio, sulfinyl, sulfonyl, imino or lower alkylimino, $alk_1$ is lower alkylene, $R_2$ is hydrogen, lower alkyl or lower alkenyl and $alk_2$ is lower alkylene, and a salt thereof, in particular a pharmaceutically acceptable salt thereof.

**4.** A process according to claim 1 for the preparation of a compound of the formula I in which $R_1$ is a phenyl radical which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, sulfonyl, imino or $C_1$-$C_4$alkylimino, $alk_1$ is $C_2$-$C_6$alkylene, $R_2$ is hydrogen or $C_1$-$C_4$alkyl and $alk_2$ is $C_2$-$C_4$alkylene, and a salt thereof.

**5.** A process according to claim 1 for the preparation of a compound of the formula I in which $R_1$ is a pyridyl or pyrimidinyl radical which is unsubstituted or substituted by $C_1$-$C_4$alkyl and/or phenyl which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, imino or $C_1$-$C_4$alkylimino, $alk_1$ is $C_2$-$C_6$alkylene, $R_2$ is hydrogen or $C_1$-$C_4$alkyl and $alk_2$ is $C_2$-$C_4$alkylene, and a salt thereof.

**6.** A process according to claim 2 for the preparation of a compound of the formula I in which $R_1$ is a phenyl, naphthyl, pyridyl or pyrimidinyl radical which is unsubstituted or monosubstituted or disubstituted by $C_1$-$C_4$alkyl, $C_1$-$C_4$alkoxy, $C_1$-$C_4$alkylenedioxy, hydroxyl, halogen having an atomic number up to and including 35 and/or trifluoromethyl, n is 0, 1 or 2, X is oxy, thio, sulfonyl, imino or $C_1$-$C_4$alkylimino, $alk_1$ is $C_2$-$C_6$alkylene, $R_2$ is $C_1$-$C_4$alkyl and $alk_2$ is $C_2$-$C_4$alkylene, and a salt thereof.

**7.** A process according to claim 1 for the preparation of a compound of the formula I in which $R_1$ is phenyl which is unsubstituted or substituted by $C_1$-$C_4$alkyl, $C_1$-$C_4$alkoxy, hydroxyl, halogen having an atomic number up to and including 35 or trifluoromethyl, or $R_1$ is unsubstituted pyridyl or pyrimidinyl, n is 0, X is oxy, thio, sulfonyl, imino or methylimino, $alk_1$ is $C_2$-$C_6$alkylene, $R_2$ is hydrogen or $C_1$-$C_4$alkyl and $alk_2$ is ethylene, and a salt thereof.

**8.** A process according to claim 2 for the preparation of a compound of the formula I in which $R_1$ is phenyl which is unsubstituted or substituted by $C_1$-$C_4$alkyl, $C_1$-$C_4$alkoxy, hydroxyl, halogen having an atomic number up to and including 35 or trifluoromethyl, or $R_1$ is unsubstituted pyridyl or pyrimidinyl, n is 0, X is oxy, thio, sulfonyl, imino or methylimino, $alk_1$ is $C_2$-$C_6$alkylene, $R_2$ is $C_1$-$C_3$alkyl and $alk_2$ is ethylene, and a salt thereof.

**9.** A process according to claim 1 for the preparation of a compound of the formula I in which $R_1$ is phenyl which is unsubstituted or substituted by $C_1$-$C_4$alkyl, $C_1$-$C_4$alkoxy or halogen having an atomic number up to and including 35, n is 0, X is oxy or thio, $alk_1$ is $C_2$-$C_4$alkylene, $R_2$ is $C_1$-$C_3$alkyl or hydrogen and $alk_2$ is ethylene, and a salt thereof.

**10.** A process according to claim 1 for the preparation of a compound of the formula I in which $R_1$ is pyridyl or pyrimidinyl which is unsubstituted or substituted by $C_1$-$C_4$alkyl or phenyl, n is 0, X is imino which is unsubstituted or substituted by $C_1$-$C_4$alkyl, or is oxy or thio, $alk_1$ is $C_2$-$C_4$alkylene, $R_2$ is $C_1$-$C_3$alkyl or hydrogen and $alk_2$ is ethylene, and a salt thereof.

**11.** A process according to claim 1 or 2 for the preparation of 3-[N-(3-phenoxypropyl)-N-methylamino]-1-hydroxypropane-1,1-diphosphonic acid or a salt thereof.

**12.** A process according to claim 1 or 2 for the preparation of 3-{N-[2-(4-methoxyphenoxy)-ethyl]-amino}-1-hydroxypropane-1,1-diphosphonic acid or a salt thereof.

**13.** A process according to claim 1 or 2 for the preparation of 3-[N-(2-phenoxyethyl)-N-methylamino]-1-hydroxypropane-1,1-diphosphonic acid or a salt thereof.

**14.** A process according to claim 1 or 2 for the preparation of 3-[N-(2-phenylthioethyl)-N-methylamino]-1-hydroxypropane-1,1-diphosphonic acid or a salt thereof.

**15.** A process according to claim 1 or 2 for the preparation of 3-[N-(3-phenoxypropyl)amino]-1-hydroxypropane-1,1-diphosphonic acid or a salt thereof.

**16.** A process according to claim 1 or 2 for the preparation of 3-[N- (3-phenylthiopropyl)-N-methylamino]-1-hydroxypropane-1,1-diphosphonic acid or a salt thereof.

**17.** A process according to claim 1 or 2 for the preparation of 3-{N-[3-(4- chlorophenoxy)propyl]-N-methylamino} -1-hydroxypropane-1,1-diphosphonic acid or a salt thereof.

**18.** A process according to claim 1 or 2 for the preparation of 3-[N-(3-(4-methoxyphenoxy)propyl)amino]-1-hydroxypropane-1,1-diphosphonic acid or a salt thereof.

**19.** A process according to claim 1 or 2 for the preparation of 3-[N-(4-(phenylthiobutyl)-N-methyl)amino]-1-hydroxypropane-1,1-diphosphonic acid or a salt thereof.

**20.** A process according to claim 1 or 2 for the preparation of 3-{N-[2-(3-trifluoromethylphenyl)amino)-ethyl]-N-methylamino}-1-hydroxypropane - 1,1-diphosphonic acid or a salt thereof.

**21.** A process according to claim 1 or 2 for the preparation of 3-{N-[3-(2-pyridylamino)-propyl]-N-methylamino}-1-hydroxypropane-1,1-diphosphonic acid or a salt thereof.

**22.** A process according to claim 1 or 2 for the preparation of 3-{N-[3-(2-pyrimidinylamino)-propyl]-N-methylamino}-1-hydroxypropane-1,1-diphosphonic acid or a salt thereof.

**23.** A process according to claim 1 or 2 for the preparation of 3-[N-(2-(benzosulfonyl)-ethyl)-N-methylamino]-1-hydroxypropane-1,1-diphosphonic acid or a salt thereof.

**24.** A process according to claim 1 or 2 for the preparation of 3-{N-[3-(4-methoxyphenoxy)-propyl]-N-methylamino}-1-hydroxypropane-1,1-diphosphonic acid or a salt thereof.

**25.** A process according to claim 1 or 2 for the preparation of 3-{N-[3-(3-methylphenoxy)-propyl]-N-methylamino}-1-hydroxypropane-1,1-diphosphonic acid or a salt thereof.

**26.** A process according to claim 1 or 2 for the preparation of 3-{N-[3-(4-fluorophenoxy)-propyl]-N-methylamino}-1-hydroxypropane-1,1-diphosphonic acid or a salt thereof.

**27.** A process according to claim 1 or 2 for the preparation of 3-[N-(4-(phenoxybutyl)-N-methyl)amino]-1-hydroxypropane-1,1-diphosphonic acid or a salt thereof.

**28.** A process according to claim 1 or 2 for the preparation of 3-[N-(6-(phenoxyhexyl)-N-methyl)amino]-1-hydroxypropane-1,1-diphosphonic acid or a salt thereof.

**29.** A process according to claim 1 or 2 for the preparation of 3-{N-[2-(4- chlorophenoxy)-ethyl]-N-methylamino}-1-hydroxypropane-1,1-diphosphonicacid or a salt thereof.

**30.** A process according to claim 1 or 2 for the preparation of 3-{{N-{3-[N'-(pyrid-2-yl)-N' -ethylamino]-propyl}-N-ethylamino}}-1-hydroxypropane-1,1-diphosphonicacid or a salt thereof.

**31.** A process according to claim 1 or 2 for the preparation of 3-{N-[2-(2-hydroxyphenoxy)-ethyl]-N-methylamino}-1-hydroxypropane-1,1-diphosphonic acid or a salt thereof.

**32.** A process for the preparation of a pharmaceutical formulation, which comprises mixing a compound obtainable according to any one of claims 1 to 31 or a pharmaceutically acceptable salt thereof with customary pharmaceutical adjuncts and excipients.

**Revendications**

**Revendications pour les Etats contractants suivants : AT, BE, CH, DE, DK, FR, GB, IT, LI LU, NL, SE**

**1.** Composés de formule I

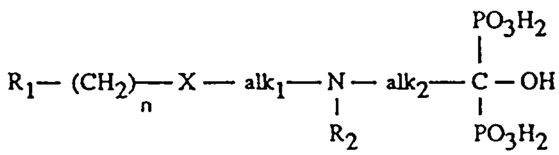

(I)

dans laquelle $R_1$ représente un reste aromatique, n représente 0, 1, 2 ou 3, X représente un groupe oxy, un groupe thio oxydé le cas échéant ou un groupe imino substitué aliphatiquement le cas échéant, $alk_1$ et $alk_2$ représentent des restes identiques ou différents bivalents et $R_2$ représente l'hydrogène, ou un groupe aliphatique monovalent, et leurs sels.

**2.** Composés selon la revendication 1 de formule I, dans laquelle $R_1$ représente un reste aromatique, n représente 0, 1, 2 ou 3, X représente un groupe oxy, un groupe thio oxydé le cas échéant ou un groupe imino substitué aliphatiquement le cas échéant, $alk_1$ et $alk_2$ représentent des restes identiques ou différents bivalents et $R_2$ représente un groupe aliphatique monovalent et leurs sels.

**3.** Composés selon la revendication 1 de formule I, dans laquelle $R_1$ représente un reste phényle, naphtyle, imidazolyle, thiazolyle, oxazolyle, pyridyle, pyrimidinyle ou pyridazinyle non substitué ou mono-, di-, ou trisubstitué par un alkyle inférieur, alcoxy inférieur, alkylènedioxy inférieur, hydroxy, halogène et/ou trifluorométhyle, notamment mono ou disubstitués, n est 0, 1, 2 ou 3, X représente un oxy, thio, sulfinyle, sulfonyle, imino ou alkylimino inférieur, $alk_1$ représente un alkylène inférieur, $R_2$ représente l'hydrogène, un alkyle inférieur ou un alcényle inférieur et $alk_2$ un alkylène inférieur et leurs sels notamment leurs sels pharmaceutiquement utilisables.

**4.** Composés selon la revendication 1 de formule I, dans laquelle $R_1$ représente un reste phényle non substitué ou mono-, ou disubstitué par un alkyle $C_1$-$C_4$, alcoxy $C_1$-$C_4$, alkylènedioxy $C_1$-$C_4$, hydroxy, halogène de numéro atomique jusqu'à 35 inclus et/ou trifluorométhyle, n est 0, 1 ou 2, X représente un oxy, thio, sulfonyle, imino ou alkylimino $C_1$-$C_4$, $alk_1$ représente un alkylène $C_2$-$C_6$, $R_2$ représente l'hydrogène ou un alkyle $C_1$-$C_4$ et $alk_2$ un alkylène $C_2$-$C_4$, et leurs sels.

**5.** Composés selon la revendication 1 de formule I, dans laquelle $R_1$ représente un reste phényle, pyridyle ou pyrimidinyle non substitué ou mono-, ou disubstitué par un alkyle $C_1$-$C_4$, alcoxy $C_1$-$C_4$, alkylène-dioxy $C_1$-$C_4$, hydroxy, halogène de numéro atomique jusqu'à 35 inclus et/ou trifluorométhyle, n est 0, 1 ou 2, X représente un oxy, thio, imino ou alkylimino $C_1$-$C_4$, $alk_1$ représente un alkylène $C_2$-$C_6$, $R_2$ représente l'hydrogène ou un alkyle $C_1$-$C_4$ et $alk_2$ un alkylène $C_2$-$C_4$ et leurs sels.

**6.** Composés selon la revendication 2 de formule I, dans laquelle $R_1$ représente un reste phényle, naphtyle, pyridyle ou pyrimidinyle non substitué ou mono-, ou disubstitué par un alkyle $C_1$-$C_4$, alcoxy

$C_1$-$C_4$, alkylènedioxy $C_1$-$C_4$, hydroxy, halogène de numéro atomique jusqu'à 35 inclus et/ou trifluorométhyle, n est 0, 1 ou 2, X représente un oxy, thio, sulfonyle, imino ou alkylimino $C_1$-$C_4$, $alk_1$ représente un alkylène $C_2$-$C_6$, $R_2$ représente un alkyle $C_1$-$C_4$ et $alk_2$ un alkylène $C_2$-$C_4$ et leurs sels.

**7.** Composés selon la revendication 1 de formule I, dans laquelle $R_1$ représente un reste phényle non substitué ou substitué par un alkyle $C_1$-$C_4$, alcoxy $C_1$-$C_4$, hydroxy halogène de numéro atomique jusqu'à 35 inclus ou trifluorométhyle ou un pyridyle ou pyrimidinyle non substitué, n est 0, X représente un oxy, thio, sulfonyle, imino ou méthylimino, $alk_1$ représente un alkylène $C_2$-$C_6$, $R_2$ représente un hydrogène ou un alkyle $C_1$-$C_4$ et $alk_2$ est l'éthylène, et leurs sels.

**8.** Composés selon la revendication 2 de formule I, dans laquelle $R_1$ représente un reste phényle non-substitué ou substitué par un alkyle $C_1$-$C_4$, alcoxy $C_1$-$C_4$, hydroxy, halogène de numéro atomique jusqu'à 35 inclus ou trifluorométhyle ou pyridyle ou pyrimidinyle non substitué, n est 0, X représente un oxy, thio, sulfonyle, imino ou méthylimino, $alk_1$ représente un alkylène $C_2$-$C_6$, $R_2$ représente un alkyle $C_1$-$C_3$ et $alk_2$ est l'éthylène ou leurs sels.

**9.** Composés selon la revendication 1 de formule I, dans laquelle $R_1$ représente un phényle non substitué ou substitué par un alkyle $C_1$-$C_4$, alcoxy $C_1$-$C_4$ ou halogène de numéro atomique jusqu'à 35 inclus, n est 0, X représente un oxy ou thio, $alk_1$ représente un alkylène $C_1$-$C_3$ ou l'hydrogène et $alk_2$ est l'éthylène, et leurs sels.

**10.** Composés selon la revendication 1 de formule I, dans laquelle $R_1$ représente un phényle, pyridyle, ou pyrimidinyle non substitué ou substitué par un alkyle $C_1$-$C_4$,n est 0, X est un imino non substitué ou substitué par un alkyle $C_1$-$C_4$, un oxy ou thio, $alk_1$ représente un alkylène $C_2$-$C_4$ et $R_2$ représente un alkyle $C_1$-$C_3$ ou l'hydrogène et $alk_2$ est l'éthylène et leurs sels.

**11.** Acide 3-[N-(3-phénoxypropyl)-N-méthyl-amino]-1-hydroxy-propane-1,1-diphosphonique ou un de ses sels.

**12.** Acide 3-[N-[2-(4-méthoxyphénoxy)-éthyl]amino]-1-hydroxy-propane-1,1-diphosphonique ou un de ses sels.

**13.** Acide 3-[N-(2-phénoxyéthyl)-N-méthyl-amino]-1-hydroxy-propane-1,1-diphosphonique ou un de ses sels.

**14.** Acide 3-[N-(2-phénylthioéthyl)-N-méthyl-amino]-1-hydroxy-propane-1,1-diphosphonique ou un de ses sels.

**15.** Acide 3-[N-(3-phénoxypropyl)-amino]-1-hydroxy-propane-1,1-diphosphonique ou un de ses sels.

**16.** Acide 3-[N-(3-phénylthiopropyl)-N-méthyl-amino]-1-hydroxypropane-1,1-diphosphonique ou un de ses sels.

**17.** Acide 3-[N-[3-(4-chlorophénoxy)-propyl]-N-méthylamino-1-hydroxy-propan-1,1-diphosphonique ou un de ses sels.

**18.** Acide 3-[N-(3-(4-méthoxyphénoxy)-propylamino]-1-hydroxy-propane-1,1-diphosphonique ou un de ses sels.

**19.** Acide 3-[N-(4-phénylthiobutyl)-N-méthyl-amino]-1-hydroxy-propane-1,1-diphosphonique ou un de ses sels.

**20.** Acide 3-[N-[2-(3-trifluorométhyl-phénylamino)éthyl]-N-méthyl-amino]-1-hydroxy-propane-1,1-diphosphonique ou un de ses sels.

**21.** Acide 3-[N-[3-(2-pyridylamino)-propyl]-N-méthyl-amino]-1-hydroxy-propane-1,1-diphosphonique ou un de ses sels.

**22.** Acide 3-[N-[3-(2-pyrimidinylamino-propyl]-N-méthyl-amino]-1-hydroxy-propane-1,1-diphosphonique ou un de ses sels.

**23.** Acide 3-[N-(2-(benzènesulfonyl)-éthyl]-N-méthyl-amino ]-1-hydroxy-propane-1,1-diphosphonique ou un de ses sels.

**24.** Acide 3-[N-[3-(4-méthoxy-phénoxy)propyl]-N-méthyl-amino]-1-hydroxy-propane-1,1-diphosphonique ou un de ses sels.

**25.** Acide 3-[N-[3-(3-méthyl-phénoxy)propyl]-N-méthyl-amino]-1-hydroxy-propane-1,1-diphosphonique ou un de ses sels.

**26.** Acide 3-[N-[3-(4-fluorophénoxy)-propyl]-N-méthyl-amino]-1-hydroxy-propane-1,1-diphosphonique ou un de ses sels.

**27.** Acide 3-[N-(4-(phénoxybutyl)-N-méthyl-amino]-1-hydroxy-propane-1,1-diphosphonique ou un de ses sels.

**28.** Acide 3-[N-(6-(phénoxyhexyl)-N-méthyl-amino]-1-hydroxy-propane-1,1-diphosphonique ou un de ses sels.

**29.** Acide 3-[N-[2-(4-chlorophénoxy)-éthyl]-N-méthyl-amino-1-hydroxy-propane-1,1-diphosphonique ou un de ses sels.

**30.** Acide 3-[[N-[3-[N'-(pyrid-2-yl)-N'-éthyl-amino] propyl]-N-éthyl-amino]]-1-hydroxy-propane-1,1-diphosphonique ou un de ses sels.

**31.** Acide 3-[N-[2-(2-hydroxy-phénoxy)éthyl]-N-méthyl-amino]-1-hydroxy-propane-1,1-diphosphonique ou un de ses sels.

**32.** Composés selon l'une des revendications 1, 3 à 5, 7, 9, 10 et 29 à 31 pour utilisation dans un procédé de traitement du corps humain ou animal.

**33.** Composés selon l'une des revendications 2, 6, 8 et 11 à 28 pour l'utilisation dans un procédé de traitement du corps humain ou animal.

**34.** Préparations pharmaceutiques comprenant un composé selon l'une des revendications 1, 3 à 5, 7, 9, 10 et 29 à 31 et/ou un de ses sels pharmaceutiquement utilisables en présence des véhicules et produits auxiliaires pharmaceutiquement utilisables.

**35.** Préparations pharmaceutiques, comprenant un composé selon l'une des revendications 2, 6, 8 et 11 à 28 ou un de ses sels pharmaceutiquement utilisables en présence des véhicules et produits auxiliaires pharmaceutiquement utilisables.

**36.** Procédé de préparation de composés de formule I

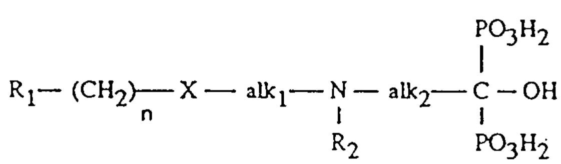

(I)

dans laquelle $R_1$ représente un reste aromatique, n représente 0, 1, 2 ou 3, X représente un groupe oxy, un groupe thio oxydé le cas échéant ou un groupe imino substitué aliphatiquement le cas échéant, $alk_1$ et $alk_2$ représentent des restes identiques ou différents bivalents et $R_2$ représente l'hydrogène ou un groupe aliphatique monovalent, et leurs sels, caractérisé en ce que

a) dans un composé de formule

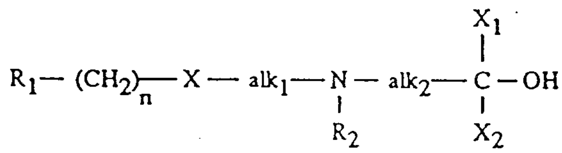

(II)

dans laquelle $R_1$, n, X, $alk_1$, $R_2$ et $alk_2$ ont les significations indiquées, $X_1$ représente un groupe phosphono à fonction transformée et $X_2$ un groupe phosphono à fonction libre ou transformée, on transforme en groupe phosphono libre le groupe phosphono à fonction transformée $X_1$ et le cas échéant $X_2$ ou

b) on fait réagir ensemble des composés de formules

$$R_1-(CH_2)_n-X-alk_1-Y_1 \quad (III)$$

et

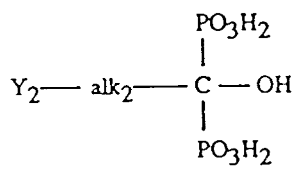

(IV),

dans lesquels l'un des restes $Y_1$ et $Y_2$ est un groupe hydroxy Y estérifié réactionnel et l'autre est un groupe de formule -N($R_2$)-H, ou leurs sels ou dans des conditions réductrices des composés de formules

$$R_1-(CH_2)_n-X-alk'_1{=}O \quad (IIIa)$$

et

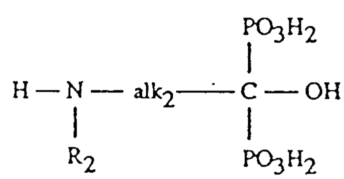

(IVa),

dans lesquelles $alk_1$' représente un reste lié par liaison double de façon appropriée au reste $alk_1$ p.ex. un reste alcanylylidène inférieur substitué de façon approprié, ou

c) on fait réagir un composé de formule

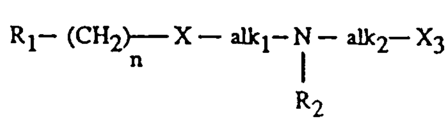

(V)

dans laquelle $X_3$ représente un carboxy, carbamyle ou cyano, avec un agent de phosphorylation, on hydrolyse le produit obtenu et dans un produit intermédiaire de formule

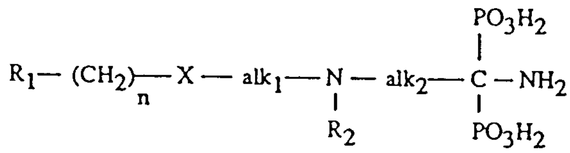

(VI)

obtenu en partant de composés de formule V, dans laquelle $X_3$ est un cyano ou un carbamyle, ou un de ses sels, on remplace le groupe amino par un hydroxy par traitement avec de l'acide nitreux ou

d) on fait réagir des composés de formules

$$R_1-(CH_2)_n-Y_3 \quad \text{(VII)}$$

et

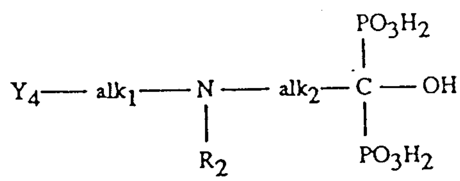

(VIII)

dans lesquelles $Y_3$ représente un groupe de formule $-X_0$-H et $Y_4$ un hydroxy estérifié mais pouvant réagir, ou, dans la mesure où n est différent de 0, $Y_3$ représente un groupe Y et $Y_4$ un groupe $-X_0$-H, $X_0$ étant un oxy, thio ou le cas échéant un imino X aliphatiquement substitué ou un imino $-NR_0$- protégé de façon provisoire, dans lequel $R_0$ est un groupe protecteur usuel d'amino, et Y est un hydroxy estérifié pouvant réagir, ou leurs sels, ou pour préparer des composés de formule I, dans laquelle X représente un groupe imino aliphatiquement substitué le cas échéant, on fait réagir dans des conditions réductrices des composés de formule

$$R_1-(CH_2)_{n-1}-CH=O \quad \text{(VIIa)}$$

et

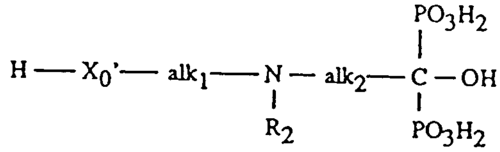

(VIIIa),

ou

$$R_1-(CH_2)_n-X_0'-H \quad \text{(VIIb)}$$

et

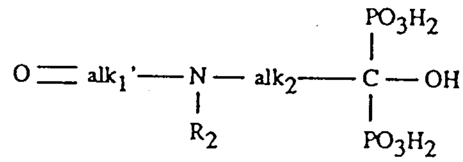 (VIIIb),

dans lesquelles $R_1$, n, $alk_1$, $R_2$ et $alk_2$ ont les significations indiquées et $X'_0$ représente également un groupe imino X aliphatiquement substitué le cas échéant ou un imino $-NR_0-$ protégé de façon provisoire, dans lequel $R_0$ est un groupe protecteur usuel d'amino, chaque fois ou élimine le groupe protecteur d'amino $R_0$, s'il est présent, et si c'est souhaité pour préparer des composés de formule I dans laquelle $R_2$ est un reste aliphatique monovalent, et leurs sels ou

e) on fait réagir des composés de formules

$R_2$-Y (IX)

et

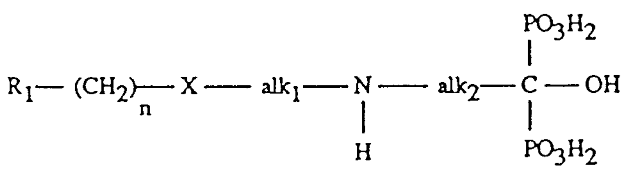 (Ia),

dans lesqelles $R_1$, n, X, $alk_1$, $R_2$ et $alk_2$ ont les significations indiquées et Y représente un hydroxy estérifié réactif, ou leurs sels ou dans des conditions réductrices des composés de formules

$R_2''=O$ (IXa)

et

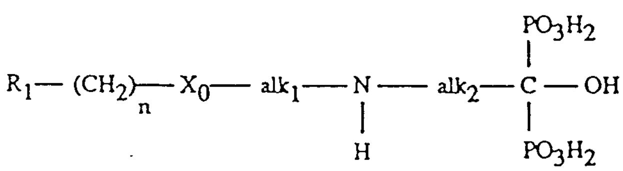 (Ia),

dans lesquelles $R_1$, n, X, $alk_1$ et $alk_2$ ont les significations indiquées, $X_0$ étant un oxy, thio ou le cas échéant un imino X substitué aliphatiquement ou un imino $-NR_0-$ protégé de façon provisoire dans lequel $R_0$ est un groupe protecteur usuel d'amino, et $R_2''$ représente un reste à liaison double correspondant au reste $R_2$, p.ex. un alkylidène inférieur, ou on transforme un composé obtenu en un autre composé de formule I, on fractionne en ses composants un mélange d'isomères qu'on obtient selon le procédé et on recueille l'isomère préféré et/ou on transforme un composé libre qu'on peut obtenir selon le procédé en un sel ou un sel fourni selon le procédé en le composé libre correspondant.

**Revendications pour les Etats contractants suivants : ES, GR**

**1.** Procédés de préparation de composés de formule I

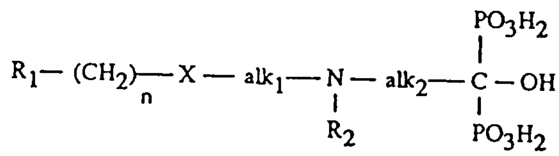

(I)

dans laquelle $R_1$ représente un reste aromatique, n représente 0, 1, 2 ou 3, X représente un groupe oxy, un groupe thio oxydé le cas échéant ou un groupe imino substitué aliphatiquement le cas échéant, $alk_1$ et $alk_2$ représentent des restes identiques ou différents bivalents et $R_2$ représente l'hydrogène ou un groupe aliphatique monovalent, et leurs sels caractérisé en ce que

a) dans un composé de formule

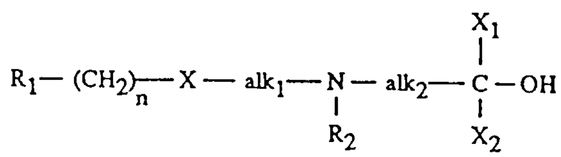

(II)

dans laquelle $R_1$, n, X, $alk_1$, $R_2$ et $alk_2$ ont les significations indiquées, $X_1$ représente un groupe phosphono à fonction transformée et $X_2$ un groupe phosphono à fonction libre ou transformée, on transforme en groupe phosphono libre le groupe phosphono à fonction transformée $X_1$ et le cas échéant $X_2$ ou

b) on fait réagir ensemble des composés de formules

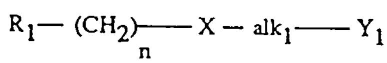

(III)

et

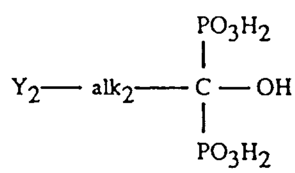

(IV),

dans lesquels l'un des restes $Y_1$ et $Y_2$ est un groupe hydroxy Y estérifié réactionnel et l'autre est un groupe de formule -N($R_2$)-H, ou leurs sels ou dans des conditions réductrices des composés de formules

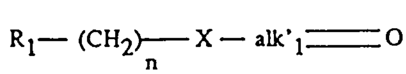

(IIIa)

et

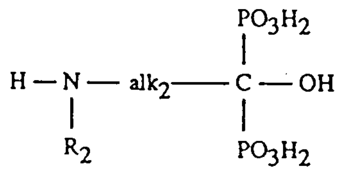 (IVa),

dans lesquelles $alk_1$' représente un reste lié par liaison double de façon appropriée au reste $alk_1$ p.ex. un reste alcanylylidène inférieur substitué de façon approprié, ou

c) on fait réagir un composé de formule

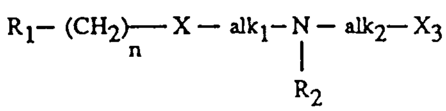 (V)

dans laquelle $X_3$ représente un carboxy, carbamyle du cyano, avec un agent de phosphorylation, on hydrolyse le produit obtenu et dans un produit intermédiaire de formule

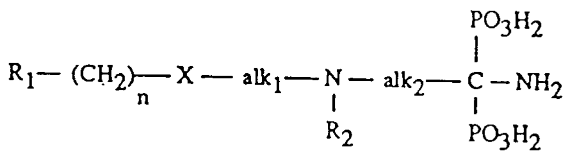 (VI)

obtenu en partant de composés de formule V, dans laquelle $X_3$ est un cyano ou un carbamyle, ou un de ses sels, on remplace le groupe amino par un hydroxy par traitement avec de l'acide nitreux ou

d) on fait réagir des composés de formules

$R_1$-($CH_2$)-$Y_3$ (VII)

et

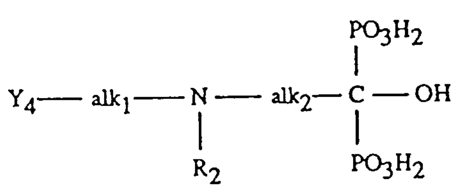 (VIII),

dans lesquelles $Y_3$ représente un groupe de formule -$X_0$-H et $Y_4$ un hydroxy estérifié mais pouvant réagir, ou, dans la mesure où n est différent de 0, $Y_3$ représente un groupe Y et $Y_4$ un groupe -$X_0$-H, $X_0$ étant un oxy, thio ou le cas échéant un imino X aliphatiquement substitué ou un imino -$NR_0$- protégé de façon provisoire, dans lequel $R_0$ est un groupe protecteur usuel d'amino, et Y est un hydroxy estérifié pouvant réagir, ou leurs sels, ou pour préparer des composés de formule I, dans laquelle X représente un groupe imino aliphatiquement substitué le cas échéant, on fait réagir dans des conditions réductrices des composés de formule

$R_1-(CH_2)_{n-1}-CH=O$ (VIIa)

et

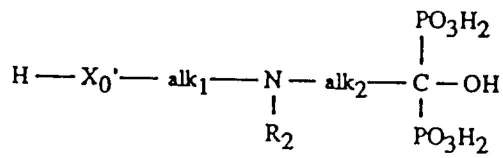

(VIIIa),

ou

$$R_1-(CH_2)_n-X_0'-H \qquad (VIIb)$$

et

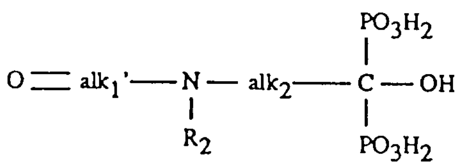

(VIIIb),

dans lesquelles $R_1$, n, $alk_1$, $R_2$ et $alk_2$ ont les significations indiquées et $X'_0$ représente également un groupe imino X aliphatiquement substitué le cas échéant ou un imino $-NR_0-$ protégé de façon provisoire, dans lequel $R_0$ est un groupe protecteur usuel d'amino, chaque fois ou élimine le groupe protecteur d'amino $R_0$, s'il est présent, et si c'est souhaité pour préparer des composés de formule I dans laquelle $R_2$ est un reste aliphatique monovalent, et leurs sels ou

e) on fait réagir des composés de formules

$R_2$-Y (IX)

et

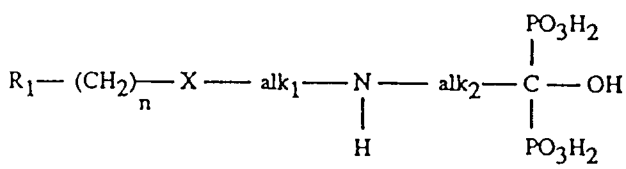

(Ia),

dans lesqelles $R_1$, n, X, $alk_1$, $R_2$ et $alk_2$ ont les significations indiquées et Y représente un hydroxy estérifié réactif, ou leurs sels ou dans des conditions réductrices des composés de formules

$R_2'' = O$ (IXa)

et

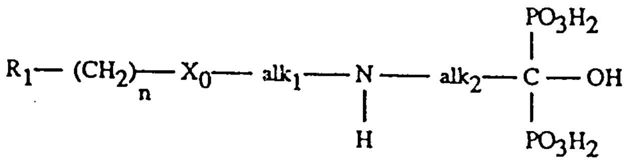 (Ia),

dans lesquelles $R_1$, n, X, $alk_1$ et $alk_2$ ont les significations indiquées, $X_0$ étant un oxy thio ou le cas échéant un imino X substitué aliphatiquement ou un imino $-NR_0-$ protégé de façon provisoire dans lequel $R_0$ est un groupe protecteur usuel d'amino, et $R_2''$ représente un reste à liaison double correspondant au reste $R_2$, p.ex. un alkylidène inférieur, ou on transforme un composé obtenu en un autre composé de formule I, on fractionne en ses composants un mélange d'isomères qu'on obtient selon le procédé et on recueille l'isomère préféré et/ou on transforme un composé libre qu'on peut obtenir selon le procédé en un sel ou un sel fourni selon le procédé en le composé libre correspondant.

**2.** Procédé selon la revendication 1 pour préparer des composés de formule I, dans laquelle $R_1$ représente un reste aromatique, n représente 0, 1, 2 ou 3, X représente un groupe oxy, un groupe thio oxydé le cas échéant ou un groupe imino substitué aliphatiquement le cas échéant, $alk_1$ et $alk_2$ représentent des restes identiques ou différents bivalents et $R_2$ représente un groupe aliphatique monovalent et leurs sels.

**3.** Procédé selon la revendication 1 pour préparer des composés de formule I, dans laquelle $R_1$ représente un reste phényle, naphtyle, imidazolyle, thiazolyle, oxazolyle, pyridyle, pyrimidinyle ou pyridazinyle non substitué ou mono-, di-, ou trisubstitué par un alkyle inférieur, alcoxy inférieur, alkylènedioxy inférieur, hydroxy, halogène et/ou trifluorométhyle, notamment mono ou disubstitués, n est 0, 1, 2 ou 3, X représente un oxy, thio, sulfinyle, sulfonyle, imino ou alkylimino inférieur, $alk_1$ représente un alkylène inférieur, $R_2$ représente l'hydrogène, un alkyle inférieur ou un alcényle inférieur et $alk_2$ un alkylène inférieur et leurs sels notamment leurs sels pharmaceutiquement utilisables.

**4.** Procédé selon la revendication 1 pour préparer des composés de formule I, dans laquelle $R_1$ représente un reste phényle non substitué ou mono-, ou disubstitué par un alkyle $C_1$-$C_4$, alcoxy $C_1$-$C_4$, alkylènedioxy $C_1$-$C_4$, hydroxy, halogène de numéro atomique jusqu'à 35 inclus et/ou trifluorométhyle, n est 0, 1 ou 2, X représente un oxy, thio, sulfonyle, imino ou alkylimino $C_1$-$C_4$, $alk_1$ représente un alkylène $C_2$-$C_6$, $R_2$ représente l'hydrogène ou un alkyle $C_1$-$C_4$ et $alk_2$ un alkylène $C_2$-$C_4$, et leurs sels.

**5.** Procédé selon la revendication 1 pour préparer des composés de formule I, dans laquelle $R_1$ représente un reste phényle, pyridyle ou pyrimidinyle non substitué ou mono-, ou disubstitué par un alkyle $C_1$-$C_4$, alcoxy $C_1$-$C_4$, alkylènedioxy $C_1$-$C_4$, hydroxy, halogène de numéro atomique jusqu'à 35 inclus et/ou trifluorométhyle, n est 0, 1 ou 2, X représente un oxy, thio, imino ou alkylimino $C_1$-$C_4$,$alk_1$ représente un alkylène $C_2$-$C_6$, $R_2$ représente l'hydrogène ou un alkyle $C_1$-$C_4$ et $alk_2$ un alkylène $C_2$-$C_4$ et leurs sels.

**6.** Procédé selon la revendication 2 pour préparer des composés de formule I, dans laquelle $R_1$ représente un reste phényle, naphtyle, pyridyle ou pyrimidinyle non substitué ou mono-, ou disubstitué par un alkyle $C_1$-$C_4$,alcoxy $C_1$-$C_4$, alkylènedioxy $C_1$-$C_4$, hydroxy, halogène de numéro atomique jusqu'à 35 inclus et/ou trifluorométhyle, n est 0, 1 ou 2, X représente un oxy, thio, sulfonyle, imino ou alkylimino $C_1$-$C_4$, $alk_1$ représente un alkylène $C_2$-$C_6$, $R_2$ représente un alkyle $C_1$-$C_4$ et $alk_2$ un alkylène $C_2$-$C_4$ et leurs sels.

**7.** Procédé selon la revendication 1 pour préparer des composés de formule I, dans laquelle $R_1$ représente un reste phényle non substitué ou substitué par un alkyle $C_1$-$C_4$, alcoxy $C_1$-$C_4$, hydroxy halogène de numéro atomique jusqu'à 35 inclus ou trifluorométhyle ou un pyridyle ou pyrimidinyle non substitué, n est 0, X représente un oxy, thio, sulfonyle, imino ou méthylimino, $alk_1$ représente un alkylène $C_2$-$C_6$, $R_2$ représente un hydrogène ou un alkyle $C_1$-$C_4$ et $alk_2$ est l'éthylène, et leurs sels.

**8.** Procédé selon la revendication 2 pour préparer des composés de formule I, dans laquelle $R_1$ représente un reste phényle non substitué ou substitué par un alkyle $C_1$-$C_4$, alcoxy $C_1$-$C_4$, hydroxy, halogène de numéro atomique jusqu'à 35 inclus ou trifluorométhyle ou pyridyle ou pyrimidinyle non substitué, n est 0, X représente un oxy, thio, sulfonyle, imino ou méthylimino, $alk_1$ représente un alkylène $C_2$-$C_6$, $R_2$ représente un alkyle $C_1$-$C_3$ et $alk_2$ est l'éthylène ou leurs sels.

**9.** Procédé selon la revendication 1 pour préparer des composés de formule I, dans laquelle $R_1$ représente un phényle non substitué ou substitué par un alkyle $C_1$-$C_4$,alcoxy $C_1$-$C_4$ ou halogène de numéro atomique jusqu'à 35 inclus, n est 0, X représente un oxy ou thio, $alk_1$ représente un alkylène $C_1$-$C_3$ ou l'hydrogène et $alk_2$ est l'éthylène, et leurs sels.

**10.** Procédé selon la revendication 1 pour préparer des composés de formule I, dans laquelle $R_1$ représente un phényle, pyridyle, ou pyrimidinyle non substitué ou substitué par un alkyle $C_1$-$C_4$, n est 0, X est un imino non substitué ou substitué par un alkyle $C_1$-$C_4$, un oxy ou thio, $alk_1$ représente un alkylène $C_2$-$C_4$ et $R_2$ représente un alkyle $C_1$-$C_3$ ou l'hydrogène et $alk_2$ est l'éthylène et leurs sels.

**11.** Procédé selon la revendication 1 ou 2 pour préparer l'acide 3-[N-(3-phénoxypropyl)-N-méthyl-amino]-1-hydroxy-propane-1,1-diphosphonique ou un de ses sels.

**12.** Procédé selon la revendication 1 pour préparer l'acide 3-[N-[2-(4-méthoxyphénoxy)-éthyl]amino]-1-hydroxy-propane-1,1-diphosphonique ou un de ses sels.

**13.** Procédé selon la revendication 1 ou 2 pour préparer l'acide 3-[N-(2-phénoxyéthyl)-N-méthyl-amino]-1-hydroxy-propane-1,1-diphosphonique ou un de ses sels.

**14.** Procédé selon la revendication 1 ou 2 pour préparer l'acide 3-[N-(2-phénylthioéthyl)-N-méthyl-amino]-1-hydroxy-propane-1,1-diphosphonique ou un de ses sels.

**15.** Procédé selon la revendication 1 pour préparer l'acide 3-[N-(3-phénoxypropyl)-amino]-1-hydroxy-propane-1,1-diphosphonique ou un de ses sels.

**16.** Procédé selon la revendication 1 ou 2 pour préparer l'acide 3-[N-(3-phénylthiopropyl)-N-méthyl-amino]-1-hydroxy-propane-1,1-diphosphonique ou un de ses sels.

**17.** Procédé selon la revendication 1 ou 2 pour préparer l'acide 3-[N-[3-(4-chlorophénoxy)-propyl]-N-méthyl-amino-1-hydroxy-propan-1,1-diphosphonique ou un de ses sels.

**18.** Procédé selon la revendication 1 pour préparer l'acide 3-[N-(3-(4-méthoxyphénoxy)-propylamino]-1-hydroxy-propane-1,1-diphosphonique ou un de ses sels.

**19.** Procédé selon la revendication 1 ou 2 pour préparer l'acide 3-[N-(4-phénylthiobutyl)-N-méthyl-amino]-1-hydroxy-propane-1,1-diphosphonique ou un de ses sels.

**20.** Procédé selon la revendication 1 ou 2 pour préparer l'acide 3-[N-[2-(3-trifluorométhyl-phénylamino)-éthyl]-N-méthyl-amino]-1-hydroxy-propane-1,1-diphosphonique ou un de ses sels.

**21.** Procédé selon la revendication 1 ou 2 pour préparer l'acide 3-[N-[3-(2-pyridylamino)-propyl]-N-méthyl-amino]-1-hydroxy-propane-1,1-diphosphonique ou un de ses sels.

**22.** Procédé selon la revendication 1 ou 2 pour préparer l'acide 3-[N-[3-(2-pyrimidinylamino-propyl]-N-méthyl-amino]-1-hydroxy-propane-1,1-diphosphonique ou un de ses sels.

**23.** Procédé selon la revendication 1 ou 2 pour préparer l'acide 3-[N-(2-(benzènesulfonyl)-éthyl]-N-méthyl-amino]-1-hydroxy-propane-1,1-diphosphonique ou un de ses sels.

**24.** Procédé selon la revendication 1 ou 2 pour préparer l'acide 3-[N-[3-(4-méthoxy-phénoxy)propyl]-N-méthyl-amino] -1-hydroxy-propane-1,1-diphosphonique ou un de ses sels.

25. Procédé selon la revendication 1 ou 2 pour préparer l'acide 3-[N-[3-(3-méthyl-phénoxy)propyl]-N-méthyl-amino]-1-hydroxy-propane-1,1-diphosphonique ou un de ses sels.

26. Procédé selon la revendication 1 ou 2 pour préparer l'acide 3-[N-[3-(4-fluorophénoxy)-propyl]-N-méthyl-amino]-1-hydroxy-propane-1,1-diphosphonique ou un de ses sels.

27. Procédé selon la revendication 1 ou 2 pour préparer l'acide 3-[N-(4-(phénoxybutyl)-N-méthyl-amino]-1-hydroxy-propane-1,1-diphosphonique ou un de ses sels.

28. Procédé selon la revendication 1 ou 2 pour préparer l'acide 3-[N-(6-(phénoxyhexyl)-N-méthyl-amino]-1-hydroxy-propane-1,1-diphosphonique ou un de ses sels.

29. Procédé selon la revendication 1 ou 2 pour préparer l'acide 3-[N-[2-(4-chlorophénoxy)-éthyl]-N-méthyl-amino-1-hydroxy-propane-1,1-diphosphonique ou un de ses sels.

30. Procédé selon la revendication 1 ou 2 pour préparer l'acide 3-[[N-[3-[N'-(pyrid-2-yl)-N'-éthyl-amino ] propyl]-N-éthyl-amino]]-1-hydroxy-propane-1,1-diphosphonique ou un de ses sels.

31. Procédé selon la revendication 1 ou 2 pour préparer l'acide 3-[N-[2-(2-hydroxy-phénoxy)éthyl]-N-méthyl-amino]-1-hydroxy-propane-1,1-diphosphonique ou un de ses sels.

32. Procédé de production de préparations pharmaceutiques caractérisé en ce qu'on mélange un composé qu'on peut obtenir selon l'une des revendications 1 à 32 ou un de ses sels pharmaceutiquement utilisables avec les véhicules et produits auxiliaires pharmaceutiquement usuels.